# EUROPEAN PATENT APPLICATION

(11) **EP 4 163 635 A1**
(43) Date of publication of application: **12.04.2023**
(21) Application number: 22182419.6
(22) Date of filing: 16.06.2020
(51) Int. Cl.: G01N 33/66, C12Q 1/26

(54) **SENSING PLATFORM**

(30) Priority: 21.06.2019 LU 101273
(62) Divisional of application: 20732599.4
(71) Applicant: ECOLE POLYTECHNIQUE FEDERALE DE LAUSANNE (EPFL), 1015 Lausanne (CH)
(72) Inventor: BOGHOSSIAN, Ardemis Anoush, 1015 Lausanne (CH); ZUBKOVS, Vitalijs, 1015 Lausanne (CH); SCHÜRGERS, Nils, 1015 Lausanne (CH)
(74) Representative: Biotronik Corporate Services SE

(57) **Abstract**

The present invention relates to a glucose sensor comprising a mutated biomolecule immobilised to a substrate, wherein the mutated biomolecule is immobilised to the substrate via a linker comprising a thiol reactive group and a substrate adherence group, wherein the biomolecule is mutated to introduce one or more surface accessible thiol moieties, and wherein the mutated biomolecule is connected to the linker via said one or more surface accessible thiol moieties on the biomolecule and said thiol reactive group on the linker, and wherein the biomolecule is glucose oxidase, concanavalin A, or glucose binding protein.

## Description

### Field of the Invention

The present invention is directed to novel sensors, particularly sensors useful in the detection of bio-analytes.

### Background to the Invention

Biosensors are analytical devices which convert a biological response into a measurable electrical signal. Biosensors may be used, for example, to determine the concentration of substances or other parameters in biological material. Biosensors find use across a range of industries, predominantly the health-care industry, but also in other areas such as food quality monitoring, environmental monitoring and the like.

A particular area of focus is the use of biosensors in the health-care industry to analyse biological samples to assist in clinical management. For example, according to the WHO, the global prevalence of diabetes among adults over 18 years of age was 8.5% in 2014 and there is a significant unmet medical need for rapid, accurate and simple glucose monitoring biosensors.

Biosensors should ideally possess some or all of the following attributes: accurate, precise, reproducible, specific, stable and cheap. There remains a significant unmet need for biosensors achieving some or all of these aims.

### Summary of the Invention

In a first aspect of the present invention there is provided a sensor B-L-S; wherein: B is a biomolecule comprising one or more surface-exposed thiol moieties, L is a linker comprising a thiol reactive group, and S is a substrate; wherein L is covalently linked to B via the one or more surface-exposed thiol moieties.

By linking the biomolecule to the linking group via a surface-exposed thiol moiety, it is possible to selectively and precisely control conjugation leading to sensors with enhanced properties. The present invention makes use of thiol reactive compounds: surface exposed on the biomolecule and on a linker to enable selective conjugation of the biomolecule and linker.

In a further aspect of the present invention there is provided a sensor comprising a biomolecule immobilised to a substrate; wherein the biomolecule is immobilised to the substrate via a linker; and wherein the biomolecule has one or more surface accessible thiol moieties and is connected to the linker via said one or more surface accessible thiol moieties.

Preferably the biomolecule is a modified biomolecule and the modification introduces said surface accessible thiol moieties. For example, pre-modification, the biomolecule may not contain any surface accessible thiol moieties but modification introduces linkable surface accessible thiol moieties. The modification may be a mutation.

As such, in a further aspect of the present invention there is provided a sensor comprising a mutated biomolecule immobilised to a substrate; wherein the biomolecule is immobilised to the substrate via a linker; wherein the biomolecule is mutated to introduce one or more surface accessible thiol moieties; and wherein the biomolecule is connected to the linker via said one or more surface accessible thiol moieties.

In a further aspect of the present invention there is provided a sensor comprising a biomolecule immobilised to a substrate via a linker, wherein the linker is connected to the biomolecule through a thiol linkage, and wherein the substrate is a material having optical or charge carrying properties. In embodiments, the thiol linkage is between one or more surface accessible thiol moieties on the biomolecule and thiol reactive group(s) on the linker.

In a further aspect of the present invention there is provided a sensor comprising a biomolecule immobilised to a carbon nanomaterial via a linker, wherein the linker is connected to the biomolecule through a thiol linkage.

In a yet further aspect of the present invention there is provided a method of controlling biomolecules immobilised on a carbon nanomaterial substrate, said method comprising: modifying said biomolecules to create biomolecules having a surface accessible thiol moieties, and immobilising said modifying biomolecules to said substrate using linkers having thiol reactive groups, wherein the biomolecules are connected to the linkers via the surface accessible thiol moieties and the thiol reactive groups.

The method enables controlled immobilisation by controlling where the linker attaches to the biomolecule. This in turn controls how the biomolecule immobilises onto the substrate since it is the linker which adheres to the substrate surface. Preferably a biomolecule will have one surface accessible thiol moiety and therefore one linker but it is possible for a biomolecule to have more than one surface accessible thiol moiety if desired.

By using a thiol based linking strategy it is possible to control at which location the linking group will attach to the biomolecule. Thiol groups are not common on protein surfaces, and therefore providing a surface-exposed thiol moiety allows for consistent placement of the linking group on the protein surface. This can, for example, ensure that the linker is place in a region such that it will not adversely affect the reactive binding site for the biomolecule. The linker may, for example, be located to facilitate charge flow through the sensor to improve sensitivity. Controlled placement may also help ensure consistent protein folding on the sensor. Controlled placement may enable greater stability of the sensor device.

The sensor may contain a plurality of biomolecules adhered to the surface of the substrate. By controlling the linking site on the biomolecule it is possible to ensure a more consistent orientation of biomolecules on the surface. Such an approach may improve density of biomolecule adherence. Improving biomolecule density and/or orientation consistency may improve the sensitivity and/or reliability of the sensor.

In comparison to directly adhering the biomolecule to the substrate, the biomolecules according to the present invention may be more stably secured to the substrate due to the linking strategy. Thy may also be applied with a higher density. They may also enable the biomolecule to better conform to its natural structure (e.g. better protein folding). Many of the same advantages may be present when compared against biomolecules linked via, say, an amide or carboxylic acid group on the biomolecule. While these linkages are possible they are less controlled than a thiol linking strategy as there are many more amide sites on the surface of a biomolecule. This leads to less control of the linking strategy and many of the same issues outlined above.

In a yet further aspect of the present invention there is provided: a glucose oxidase (GOx) polypeptide comprising one or more mutations, a glucose-binding protein (GBP) comprising one or more mutations, a concanavalin A (ConA) comprising one or more mutations, a cholesterol oxidase comprising one or more mutations, a carbohydrate oxidase polypeptide comprising one or more mutations or a lactate oxidase polypeptide comprising one or more mutations; wherein said one or more mutations introduces a site-selective thiol conjugation site selected to permit conjugation of the polypeptide to a linker comprising a thiol reactive group. Such modified polypeptides enables sensors with enhanced properties.

In a yet further aspect of the present invention, there is provided a polynucleotide encoding the glucose oxidase polypeptide, the carbohydrate oxidase polypeptide, or the lactate oxidase polypeptide of the present invention.

In a yet further aspect of the present invention, there is provided a nucleic acid construct comprising the polynucleotides of the present invention operably linked to a regulatory sequence.

In a yet further aspect of the present invention there is provided a method of producing a sensor, comprising:
a) providing a biomolecule comprising one or more surface-exposed thiol moieties;
b) contacting the biomolecule with a linker having a thiol reactive group and covalently linking the biomolecule and linker;
c) contacting the conjugated biomolecule-linker with a substrate such that the biomolecule-linker adheres to the surface of the substrate.

The method may involve modifying the biomolecule to obtain one or more surface-exposed thiol moieties.

Preferably, the method involves a surfactant exchange method.

In a yet further aspect of the present invention, there is provided the use of a sensor according to the present invention in the detection and/or quantification of an analyte in a sample. In a yet further aspect there is provided a method of detecting and/or quantifying an analyte in a sample comprising the use of a sensor according to the present invention. The method may comprise contacting the sample with a sensor according to the present invention and detecting a change in the sensor properties due to presence of the analyte. The analyte may be, for example, the glucose level in a patient.

In a yet further aspect of the present invention, there is provided a method of attaching a biomolecule to a carbon nanomaterial substrate comprising: modifying the biomolecule to introduce one or more surface-exposed thiol moieties; attaching a linker to the biomolecule wherein the linker comprises a thiol reactive group and a substrate adherence group; wherein the linker attaches to the biomolecule via the thiol group and thiol reactive group; and wherein the biomolecule-linker adheres to the substrate via the substrate adherence group.

In a yet further aspect of the present invention, there is provided a method of obtaining structured immobilisation of biomolecules on a substrate comprising modifying the biomolecules to comprise one or more surface-exposed thiol moieties; attaching linking groups to the biomolecules via the surface-exposed thiol moieties and a thiol reactive group on the linking group; and adhering the biomolecules to the substrate via the linking groups.

### Description of the Drawings

Figure 1 shows a GOx monomer crystal structure with selected amino acids for point mutations. The illustration show amino acid residues K13, D70, A418, and H446 (blue) and the glucose binding cavity with FAD co-factor (red) (distances between the glucose-binding cavity and the mutated residues are indicated with dashed lines).
Figure 2 shows a 0.7% agarose gel of recombinant plasmids for GOx expression (90 V, 40 minutes). The gel was stained using a SYBR Gold dye.
Figure 3a shows elution fraction which were obtained in purification of the GOx variants by size-exclusion chromatography. Absorbance of proteins in the eluted fractions was monitored at 280 nm. Presence of GOx in the eluted fractions was identified in a colorimetric ABTS GOx activity assay (peak of GOx is labelled on the graph).
Figure 3b shows SDS-PAGE analysis of cell culture media obtained in the following GOx purification steps: (b1) GOx expression media; (b2) the media after concentration; (b3) the media before and (b4) after size-exclusion chromatography purification. A protein marker IV was used as the reference (AppliChem).
Figure 4 shows SDS-PAGE analysis of purified GOx (lanes 2 - 7) from *P. pastoris* compared with the commercial wild type GOx from *A. niger* (lane 1) (denoted as WT*). The wild type (WT) GOx was shown after two different protein expressions (lanes 2 and 3). The 15% sodium dodecyl sulphate polyacrylamide gel stained with the Coomassie Brilliant Blue dye. A protein marker IV was used as the reference (AppliChem).
Figure 5 shows circular dichroism (CD) spectra of GOx variants from *P. pastoris* and a commercial WT* GOx from *A. niger.*
Figure 6 shows fitted CD spectra of GOx variants using a secondary structure prediction software BeStSel (Micsonai, A., et al. Accurate secondary structure prediction and fold recognition for circular dichroism spectroscopy. Proceedings of the National Academy of Sciences. 112, E3095-E3103 (2015); Micsonai, A., et al. BeStSel: A web server for accurate protein secondary structure prediction and fold recognition from the circular dichroism spectra. Nucleic Acids Research. 46, W315-W322 (2018)).
Figure 7 shows the distribution of BeStSel-predicted percentage of structure features (a-helixes and β-strands) in the GOx variants. The variants are compared to the crystal structure from the PDB 1CF3 code. A star symbol indicates the sample which was made of commercial GOx obtained from *A. niger.*
Figure 8 shows relative activity rates of GOx towards glucose as measured by the ABTS colorimetric assay. The enzyme was omitted in the control sample. The error bars indicate standard deviations among technical triplicates.
Figure 9 shows UV-Vis absorption spectra after addition of SPDP to the GOx solutions measured at 1, 8, 30, 60, and 120 minutes time points. Spontaneous degradation of SPDP was measured in PBS (control). The absorbance spectra measured before addition of SPDP were subtracted for each measurement.
Figure 10 shows changes in absorbance at 343 nm for GOx variants measured before and after addition of SPDP. The control sample contains the reaction mix without GOx.
Figure 11 is an illustration of GOx-PM-SWCNT preparation steps. (1) Reduction of a GOx mutant and its conjugation to PM; (2) Purification of GOx-PM from unreacted PM; (3) Wrapping of SC-suspended SWCNTs with PM-GOx and dialysis of SC; (4) Removing of free GOx in the final dialysis step.
Figure 12 shows UV-Vis absorbance spectra of GOx (green line), PM (blue line), and GOx-PM (orange line). The absorption of aromatic amino acids of the protein is seen at 280 nm. The broad peak at 450 nm corresponds to FAD absorption. The spectrum of a PM crosslinker show peaks at 268, 278, 315, 329, and 345 nm. GOx concentration in the protein samples was 0.5 mg mL⁻¹. The dotted line represents the PM absorbance peak maximum at 345 nm.
Figure 13 shows emission spectra of GOx(WT) (green line) and GOx(70C)-PM (orange line) upon PM excitation at 345 nm. The GOx concentration in the samples was 0.5 mg mL⁻¹. The dotted lines represent GOx-PM fluorescence peak maxima of a pyrene at 376 and 396 nm, and two pyrenes in close proximity at 480 and 495 nm.
Figure 14 shows UV-Vis-NIR absorbance spectra of GOx-PM-SWCNTs (red line) in comparison to the suspension obtained with the non-specifically adsorbed GOx(WT)-SWCNTs (purple line), and SC-SWCNT (blue line). The spectra were normalized to SWCNT absorbance at 739 nm. The inserts show absorbance spectra of the merged *E₂₂* (7,5), *E₂₂* (7,6) SWCNT peaks between 620 and 700 nm, and the *E₁₁* (7,6) SWCNT peak between 1080 and 1190 nm. The dotted line at 345 nm represents a position of the PM absorbance peak.
Figure 15 shows normalized near-infrared fluorescence spectra of GOx-PM-SWCNTs (red line) and GOx(WT)-SWCNTs (purple line). Excitation at 660±5 nm. The dotted lines represent GOx-PM-SWCNT fluorescence peak maxima of the *E₁₁* (7,5) and *E₁₁* (7,6) SWCNTs at 1050 and 1153 nm, respectively.
Figure 16a shows the relative activity rates of GOx towards glucose measured in an ABTS colorimetric assay. The activity rates of initial GOx/SC-SWCNTs suspensions (red), as well after 24 h (green) and 48 h (blue) of dialysis in 300 kDa MWCO devices. The enzyme was omitted in the (0) sample. Samples (i), (ii), and (iii) had initial concentrations of GOx 0.3, 0.9, and 2.8 mg mL⁻¹. The sample (+) GOx-PM-SWCNT was obtained after the final dialysis step (initial protein concentration was 1.4 mg mL⁻¹).
Figure 16b shows images of the suspensions taken before and after the dialysis. After the dialysis, the samples were centrifuged to precipitate aggregated SWCNTs.
Figure 17a shows absorbance measured (at 414 nm) in the ABTS colorimetric activity assay for GOx-SWCNTs with initial concentrations 0.3, 0.9, and 2.8 mg mL⁻¹. Data was fitted with linear functions and obtained slope values listed in the legend.
Figure 17b shows spectroscopically-measured GOx concentrations vs. determined relative GOx activity rates.
Figure 18 shows NIR fluorescence spectra of the GOx-PM-SWCNTs (blue line) and SC-SWCNTs (purple line) before (dashed lines) and 2 minutes after addition of 20 mM glucose (solid line). The *E₁₁* (7,6) peak was normalized to its maximum of the fluorescence intensity before addition of the glucose solution. Excitation at 660±5 nm.
Figure 19 shows the *E₁₁* (7,6) peak intensity monitoring of the GOx-PM-SWCNTs in a device with a glucose-permeable membrane. 20 mM glucose in PBS was added on the top of the membrane and washed with PBS after the fluorescence reached a plateau.

The fluorescence (/) was normalized to the intensity at 0 minutes *(l₀*) as *(l-l₀)*/*l₀.* Excitation at 660±5 nm.

### Detailed Description of the Invention

The following embodiments apply to all aspects of the present invention.

"Biomolecule" (B) may be any peptide, polypeptide, protein, antibody or the like which is used to bind the target analyte. Suitable biomolecules include, but are not limited to, glucose oxidase, concanavalin A, glucose binding protein, cholesterol oxidase, lactate oxidase and the like. A preferred biomolecule is glucose oxidase (GOx).

The biomolecule comprises one or more surface-exposed thiol moieties.

"Surface-exposed" means that the thiol moiety is accessible to the thiol reactive linking group such that the linker can attach to the biomolecule via the exposed thiol group. The thiol moiety is therefore surface accessible.

"Thiol moiety" means a moiety which contains a thiol group. A suitable thiol moiety is a cysteine residue. Further suitable thiol moieties include non-natural amino acids containing a thiol group. An example of a non-natural amino acid containing a thiol group is selenocysteine. Since pairs of thiol groups in proteins typically form disulfide bonds either within the same biomolecule or between biomolecules, it may be necessary to reduce the biomolecule to form free thiol groups suitable for linking.

In a preferred embodiment, the biomolecule comprises a single surface accessible thiol moiety. In an alternative embodiment the biomolecule comprises a plurality of surface accessible thiol moieties.

Having a single surface accessible moiety ensures each biomolecule orients in the same direction. In some embodiments, however, it may be advantageous to have more than one linking point. Having multiple linkers may increase the binding strength of the biomolecule to the substrate which may, for example, improve resilience of the device. Further, it may be possible to better control orientation of the biomolecule on the surface by having multiple tether points. It could, for example, ensure a particular protein presentation or folding conformation.

In a preferred embodiment, the biomolecule is modified to provide a surface exposed thiol moiety. By this approach, it is possible to fully control the linking position. However, in some instances, the biomolecule may naturally contain one or more surface exposed thiol moieties which are suitable as a linking position for the linker. Thus, in some embodiments, it is not necessary to modify the biomolecule for use in the present invention. However, in a preferred embodiment, the biomolecule is modified.

"Linker" or "linking group" means a group which attaches to the biomolecule and enables the biomolecule-linker to adhere to the substrate. Suitable linkers comprise a group which enables attachment to the biomolecule and a group which enables attachment to the substrate. The linker is preferably covalently attached to the biomolecule via a thiol reactive group. The linker is preferably non-covalently adhered to the surface of the biomolecule. The linker therefore comprises a thiol reactive group and a substrate adherence group. In an embodiment, the linker is a hydrophobic linker. In an embodiment, the linker does not comprise DNA. In an embodiment the linker comprises monomeric molecules. In a preferred embodiment, the linker is a hydrophobic linker with thiol reactive group(s) and is not DNA.

"Thiol reactive group" means a group which can form bonds with a thiol group. Suitable groups include maleimides, aziridines, acryloyl derivatives, arylating agents, pyridyl dusulfides, disulfide reagents, vinylsulfone derivatives, haloacetyl and alkyl halide derivatives (including iodoacetyl and bromoacetyl and the like). A preferred thiol reactive group is maleimides.

"Substrate adherence group" means a group which is configured to adhere to the substrate. This may be via non-covalent bonds, for example π-π stacking. Suitable substrate adherence groups include, but are not limited to, pyrenyl, porphyrin, triphenylene, anthracene, dibenzocyclooctyne, pentacene, naphthalene. In an embodiment, the substrate adherence group comprises at least one aromatic ring. In a further embodiment, the substrate adherence group comprises at least two, at least three or at least four aromatic rings.

As such, the linker comprises a moiety which can form bonds with a thiol group on the biomolecule, and a moiety which enables adherence to the substrate. A particular linker according to the present invention comprises a maleimide group as thiol reactive group, and a pyrenyl group as substrate adherence group. A particular linker is N-(1-pyrenyl)-maleimide (PM).

The size of the linker will depend on the requirements of sensor. In embodiments, the linker length will be selected to allow the biomolecule to adopt the correct conformational structure on the surface of the substrate. In further embodiments, the linker length will be selected to facilitate charge transfer from the reactive site on the biomolecule to the substrate.

In an embodiment, the linker length is selected to immobilise the biomolecule within about 50 Angstrom from the substrate surface, preferably below about 25 Angstrom, below about 15 Angstrom, below about 10 Angstrom, below about 7 Angstrom, or below about 5 Angstrom. Particularly preferred is below about 15 Angstrom to facilitate direct electron transfer. In an embodiment, the linker length is selected to immobilise the reactive site of the biomolecule within the aforesaid distance from the substrate surface.

The linker may comprise the thiol reactive group directly linked to the substrate adherence group. Alternatively, the linker may include a spacer between the thiol reactive group and the substrate adherence group. Suitable spacers include alkyl, alkenyl or alkynyl groups, cycloalkyls, cycloalkenyls, , or heterocyclic groups, which may optionally be substituted by one or more suitable substituents. Suitable spacers also include polymer chains, for example polyethylene glycol, polypropylene glycol, polytetramethylene glycol or the like. Short polymer chains are preferred.

"Substrate" means a material upon which the biomolecule is immobilised. Suitable substrates include carbon nanomaterial, including nanocarriers, single-walled carbon nanotubes (SWCNTs), multi-walled carbon nanotubes (MWCNTs), single-walled carbon nanohorns, fullerene, graphene, graphene oxide and the like.

In an embodiment, the substrate is a material which has optical properties. Such a substrate may be used in an optical sensor. An example of a material with optical properties is SWCNTs which fluoresce.

In an embodiment, the substrate is a material which has charge carrying properties. Such a material can allow electron transfer onto the material. Materials with charge carrying properties can be used in electrochemical sensors. An example of a material with charge carrying properties is MWCNTs.

"Sensor" means a device which is able to detect a target condition (for example the presence of an analyte) and to provide a detectible signal which can be used to determine whether the condition is present (for example to determine the presence or concentration of said analyte). In an embodiment, the sensor is an optical sensor. In a further embodiment, the sensor is an electrochemical sensor. In a preferred embodiment, the sensor is an optical sensor.

The present invention provides biomolecules immobilised on a substrate. In a preferred embodiment, the biomolecules are aligned on the substrate due to the positioning of the linking group. Such a conformance can increase packing density and/or sensitivity. The degree of alignment will depend on the substrate and the linking strategy.

The present invention provides a mutated biomolecule; wherein said mutation introduces one or more site-selective thiol conjugation site selected to permit conjugation of the polypeptide to linker comprising a thiol reactive group. Preferably, the one or more mutations are not in the analyte-binding cavity of the polypeptide. According to the present invention, the term "mutation" or "mutated" does not include a C-terminus modification to the biomolecule. Said another way, a biomolecule including a C-terminus modification to introduce a thiol reactive group would not fall within the scope of the present invention.

The present invention provides: a glucose oxidase polypeptide comprising one or more mutations, a carbohydrate oxidase polypeptide comprising one or more mutations or a lactate oxidase polypeptide comprising one or more mutations; wherein said one or more mutations introduces a site-selective thiol conjugation site selected to permit conjugation of the polypeptide to linker comprising a thiol reactive group. Preferably, the one or more mutations are not in the analyte-binding cavity of the polypeptide.

When determining suitable mutation positions, a number of considerations may be taken into account. First, the mutated residue must be surface-exposed. Second, to facilitate charge transfer, if the biomolecule is an enzyme, the distance between the mutated residue and the active site of the protein should be short to facilitate charge transfer onto the substrate. Third, the mutated residue should not adversely interfere with the active site of the biomolecule. Fourth, if the protein activity is based on an allosteric effect, e.g. a conformational change in the biomolecule (for example glucose binding protein), then the mutation should ideally be implemented at the flexible domain (or domains).

Preferably the mutation is a substitution. Preferably the substitution is a substitution of the wild type residue for a residue that is suitable for conjugation. Preferably said residue is cysteine, but alternatively the residue is a non-natural amino acid including a thiol or selenol group. An example of an alternative amino acid is selenocysteine.

In one embodiment, the polypeptide is glucose oxidase (GOx). In an embodiment, the wild type (i.e. non-mutated) sequence of GOx comprises or consists of SEQ ID NO: 1 or a functional variant thereof. The present invention relates to one or more substitutions in the wild type sequence. In one embodiment, the substitution is at one or more of the following positions in SEQ ID NO: 1: 13, 70, 418 and 446 or any combination thereof. In a further preferred embodiment, the substitution is one or more of the following: K13C, D70C, A418C and H446C or any combination thereof.

In one embodiment, the sequence of the GOx polypeptide is selected from SEQ ID NO: 2, 3, 4, 5 or 6 or a functional variant thereof wherein X is selected from a cysteine or an amino acid including a thiol or selenol group, for example selenocysteine.

The term "functional variant" as used herein with reference to any of SEQ ID NOs: 1 to 6 refers to a variant sequence or part of the sequence which retains the biological function of the full non-variant sequence. In the context of SEQ ID Nos 1 to 6, this may mean that the variant sequence is able to catalyse the oxidation of glucose to hydrogen peroxide and D-glucono-delta-lactone. A functional variant also comprises a variant which has sequence alterations that do not affect function, for example in non-conserved residues. Also encompassed is a variant that is substantially identical, i.e. has only some sequence variations, for example in non-conserved residues, compared to the wild type sequences as shown herein and is biologically active. Each of the proposed modifications is well within the routine skill in the art, as is determination of retention of biological activity of the encoded products.

As used in any aspect of the invention described herein a "variant" or a "functional variant" has at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99% overall sequence identity to the non-variant amino acid sequence.

Two nucleic acid sequences are said to be "identical" if the sequence of amino acids in the two sequences is the same when aligned for maximum correspondence as described below. The terms "identical" or percent "identity," in the context of two or more amino acids, refer to two or more sequences or subsequences that are the same or have a specified percentage of amino acids that are the same, when compared and aligned for maximum correspondence over a comparison window, as measured using one of the following sequence comparison algorithms or by manual alignment and visual inspection. For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Non-limiting examples of algorithms that are suitable for determining percent sequence identity and sequence similarity are the EMBOSS Stretcher algorithms.

In another aspect of the invention, there is provided an isolated polynucleotide encoding a GOx polypeptide of the present invention. In one embodiment, the polynucleotide encodes a modified GOx polypeptide as defined in any one of SEQ ID NO: 2 to 5 or a functional variant thereof. In a further embodiment, the GOx polypeptide comprises or consists of a sequence selected from SEQ ID NO: 8 to 11 or a functional variant thereof.

In another aspect of the invention there is provided a nucleic acid construct comprising a polynucleotide of the present invention operably linked to a regulatory sequence. Preferably the nucleic acid construct is an expression vector, for example pBSYA3Z. Also provided is a host cell comprising the nucleic acid construct.

The term "operably linked" as used herein refers to a functional linkage between the regulatory sequence and the polynucleotide of the present invention, such that the regulatory sequence is able to initiate transcription of the polynucleotide. In one embodiment, the regulatory sequence is a promoter, such as a constitutive promoter. Suitable promoters would be well known to the skilled person.

In a further aspect of the invention, there is provided a method of making the GOx polypeptide of the invention, the method comprising introducing and expressing the nucleic acid construct of the present invention in a host cell.

The present invention provides methods to manufacture sensors according to the present invention. In an embodiment, the method comprises a number of steps:
1) A mutated biomolecule is prepared to provide one or more surface exposed thiol moieties. This may involve mutation of a biomolecule. Alternatively, the biomolecule may inherently have a suitable exposed thiol moiety.
2) The biomolecule may be reduced to permit conjugation to the thiol reactive group on the linker.
3) The biomolecule-linker may optionally be purified to remove unreacted linker or other reagents and starting materials.
4) The biomolecule-linker may be adhered to the surface of the substrate.
5) The biomolecule-linker-substrate may be purified to remove free biomolecule or other reagents.

The molecules of the present invention may be useful in a range of applications. The molecules may be useful as optical sensors for analyte monitoring. Potential analytes include glucose, cholesterol, lactate. The molecules may be useful as imaging agents for imaging, for example near infrared imaging agents. The molecules may be useful as electrochemical sensors for analyte monitoring. The molecules may be useful as targeted protein-based carbon based nano-molecules (e.g. carbon nanotube) intra- or extra- cellular delivery. The molecules of the present invention may be useful for therapy. For example, the molecules may have use in a nanomedical therapy to deliver nanomaterial to a target, for example a tumour. As an example, the molecules (such as antibodies) may be used for targeted nanomaterial (such as SWCNTs) delivery to a tumour in photo-induced cancer treatment.

The invention will now be described with reference to the following non-limiting examples.

### Materials and Methods

### Construction of recombinant plasmids for GOx expression

Positions for non-conserved amino acids were identified using ConSurf software (Ashkenazy, H., et al. ConSurf 2010: calculating evolutionary conservation in sequence and structure of proteins and nucleic acids. Nucleic Acids Res. 38, 529-533 (2010)). GOx expression plasmids were constructed as using a puC57 vector harbouring a synthetic gene of *Aspergillus niger* GOx (GeneBank X16061; SEQ ID NO: 6) that lacks the signal peptide.

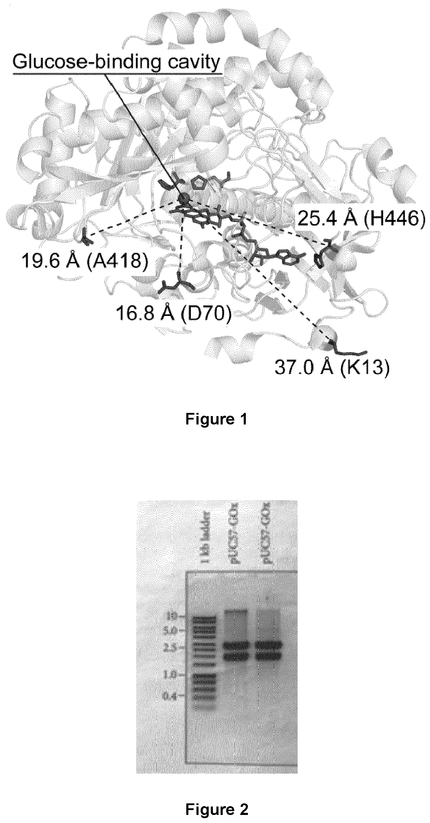

Site directed mutagenesis for the amino-acid exchanges was performed by two single primer reactions according to a protocol by Oded et al. (Edelheit, O., et al. Simple and efficient site-directed mutagenesis using two single-primer reactions in parallel to generate mutants for protein structure-function studies. BMC Biotechnol. 9, 1-8 (2009)) with the exception of Pwo polymerase, which was substituted by Q5 polymerase. The wild type and variants of the synthetic gox gene were amplified using primer pairs from Table 1 that add a Sapl restriction site, and the gene was ligated into the Sapl digested expression vector, pBSYA3S1Z. The ligation fused the coding sequence to an α-factor secretion signal. The resulting vectors were subcloned into *E. coli* and verified by sequencing (GATC, Germany). The PCR was performed in 0.2 mL MicroAmp reaction tubes (applied Biosystems, Life technologies) in a SimpliAmp Thermal Cycler (Thermo Fisher Scientific). Briefly, the 50 µL reaction mix included 5 µl of a 40 pM primer, 4 µL (or 500 ng) of the plasmid DNA, 2 µL of 0.2 mM dNTPs, 1 µl (or 1.5 U) of Q5 Polymerase, 10 µL 5x Q5 buffer from a cloning kit (M0491S, New England Biolabs), and 28 µL of ddH₂O water. The PCR protocol included denaturation for 15 s at 98°C, annealing for 15 s at 60°C, elongation for 2 h 30 min at 72°C. This procedure was repeated over 33 cycles. Next, the 40 µL of two single-primer PCR products with forward and reversed primers were combined in a PCR tube, and denatured for 5 min at 98 °C to separate the synthesized DNA from the plasmid template DNA. The tubes were gradually cooled from 98°C to 16°C. The non-mutated DNAs were digested by 1 µL of 20 U Dpnl in 2.5 µL of FD Buffer for 3 h at 37°C and incubated overnight at 10°C. The digested PCR products were analysed by agarose gel electrophoresis (Figure 2) and purified from the gel using a QIAEX II Gel Extraction Kit. Finally, we obtained -140 ng µL⁻¹ expression plasmids, as measured with a NanoDrop 2000 spectrometer (Thermo Fisher Scientific). The plasmids were stored at 20°C.

**Table 1 Synthetic double stranded DNA primers used for preparation of mutated GOx expression plasmids**

| Primer | Primer sequence (5' to 3') | Size (bp) | SEQ ID NO: |
|---|---|---|---|
| K13C-F | GGA CTG ACC CTT GTG ACG TCT CAG GTC SEQ ID NO: 12 | 27 | 12 |
| K13C-R | GAC CTG AGA CGT CAC AAG GGT CAG TCC SEQ ID NO: 13 | 27 | 13 |
| D70C-F | GAA TGC TTA TGG TTG TAT CTT CGG ATC TTC SEQ ID NO: 14 | 30 | 14 |
| D70C-R | GAA GAT CCG AAG ATA CAA CCA TAA GCA TTC SEQ ID NO: 15 | 30 | 15 |
| A418C-F | GAC CTG AGA CGT CAC AAG GGT CAG TCC SEQ ID NO: 16 | 27 | 16 |
| A418C-R | GTT CCT TGA CAC TTG TGG TGT CGC TTC SEQ ID NO: 17 | 27 | 17 |
| H446C-F | GGA CCC ATA CCT TTG TCA CTT CGC TTA CG SEQ ID NO: 18 | 29 | 18 |
| H446C-R | CGT AAG CGA AGT GAC AAA GGT ATG GGT CC SEQ ID NO: 19 | 29 | 19 |

### P. pastoris transformation

Prior to transformation, *P. pastoris* BG11 competent cells were prepared according to a protocol reported by J. Lin-Cereghino et al. (Lin-Cereghino, J. et al. Condensed protocol for competent cell preparation and transformation of the methylotrophic yeast P. pastoris. Biotechniques 38, 44-48 (2005)). 100 µL competent *P*. *pastoris* cells were mixed with 3 µg of pBSYA3-GOx-K13C, pBSYA3-GOx-D70C, pBSYA3-GOx-A418C, pBSYA3-GOx-H446C plasmids and wild type pBSYA3-GOx plasmid. Backbone plasmid pBSYA3S1Z was obtained from Bisy e.U.

Competent *P*. *pastoris* BG11 cells were transformed with 3 µg plasmid DNA by electroporation at 1.25 kV (Eppendorf Eporator) and transformed colonies were selected on YPD agar plates with 100 µg mL⁻¹ Zeocin. 12 randomly selected clones from each strain were transferred inoculated in 250 µL of BMD 1% growth medium in a sterile 96-deep well plate covered with a gas-permeable 114 µm Rayon film (VWR International) at 30°C while shaking at 300 rpm. After three days, 250 µL of BMM2 medium was added to the wells. After 12 additional hours, 50 µL of BMM10 medium were added, and this step was repeated two times after an additional 12 and 24 h of incubation. The media preparation protocols are shown in Table 2. After four days, the optical densities of the cultures was measured at 600 nm, and the cultures were centrifugated at 3220 x g for 10 minutes. The supernatants collected to test for GOx activity, and the cell pellets stored at 4°C.

**Table 2: Cell culture media used for E. coli and P. pastoris culturing**

| **Medium** | **Preparation protocols** |
|---|---|
| LB growth medium | 10 g of tryptone (Sigma), 5 g of yeast extract (Becton Dickinson and Company), and 10 g of NaCl (Sigma) dissolved in dH₂O in a 1 L bottle. Next, pH adjusted to 7.0 and the solution was sterilized in an autoclave. |
| LB growth medium and agar | Agar added to the LB growth medium at concentration 15 g L⁻¹. |
| YPD growth medium | 10 g of yeast extract, 20 g of peptone (Becton Dickinson and Company) were dissolved in 900 mL dH₂0 and autoclaved. 100 mL of sterile 220 g L⁻¹ dextrose (Sigma) were added to the solution. |
| YPD growth medium and agar | 15 g L⁻¹ of agar were dissolved in the YPD growth medium. |
| BMD 1% growth medium | In a 1 L bottle was mixed sterile solutions of 50 mL of 220 g L⁻¹ dextrose (Sigma), 200 mL of 1M K₂PO₄ buffer at pH 6, 100 ml of 134 g L⁻¹ of yeast nitrogen base w/o amino acids (Becton Dickinson and Company), and 2 mL of 200 µg mL⁻¹ Biotin (Fluka Chemia AG) filtered through a sterile 0.2 µm porous filter. The bottle filled with sterile dH₂O. Zeocin (InvivoGen-Eubio) added at concentration 100 µg mL⁻¹. |
| BMM2 (1% methanol) medium | In a 1 L bottle was mixed 10 mL of methanol (Carl Roth GmbH), 200 mL of 1M K₂PO₄ buffer at pH 6, 100 mL of 134 g L⁻¹ of yeast nitrogen base yeast nitrogen base w/o amino acids, 2 mL of 200 µg mL⁻¹ Biotin filtered through a sterile 0.2 µm porous filter. The bottle filled with sterile dH₂O. Zeocin added at concentration 100 µg mL⁻¹. |
| BMM10 (5% methanol) medium | In a 1 L bottle was mixed 50 mL of methanol, 200 mL of 1M K₂PO₄ buffer at pH 6, 200 mL of 134 g L⁻¹ of yeast nitrogen base yeast nitrogen base w/o amino acids, 2 mL of 200 µg mL⁻¹ Biotin filtered through a sterile 0.2 µm porous filter. The bottle filled with sterile dH₂O. Zeocin added at concentration 100 µg mL⁻¹. |

### Colorimetric GOx enzymatic activity assay

Enzymatic activity was measured colorimetrically using ABTS (BioChemica, ITW Reagents) (Bateman, R. C., et al. Using the Glucose Oxidase/Peroxidase System in Enzyme Kinetics. J. Chem. Educ. 72, 240-241 (1995)). Briefly, 119.8 µL of 50 mM sodium citrate buffer at pH 5.75 was mixed with 80 µL of 1 M glucose (β-D-glucose, AB 136302, ABCR GmbH & CO. KG). Also, 40 µL of 20 mM ABTS prepared in the sodium citrate buffer. 0.2 µL of 2 mg mL⁻¹ horseradish peroxidase type VI (P6782, Sigma) was added before transferring the mixture to 15 µL of the cell culture supernatant. Absorbance at 414 nm was continuously measured in a plate reader Varioskan LUX. Changes in absorbance were normalized to the optical density of the original expression cultures.

### GOx expression in P. pastoris

Recombinant GOx was produced in *P*. *pastoris.* Selected GOx clones (K13C, D70C, A418C, H446C) and wild type GOx were used for protein expression in 2 L Erlenmeyer flasks with cotton tissue plugs. The cells were added to the flasks to 75 mL of BMD 1% medium, and they were incubated at 30°C while shaking at 300 rpm for two days. On the third day, 75 mL of BMM2 medium was added to induce the protein expression. After 12 h, 15 mL of BMM10 medium was added to the flask, and this step was repeated two times. The cell cultures were then centrifuged at 3220 × g for 10 minutes, and the supernatants were filtered through a 0.2 µm porous filter. The supernatants were kept on ice and concentrated down to -30 mL using a Vivaflow 50R 30 kDa MWCO crossflow dialysis device (Sartorius). The solutions were further concentrated in a Amicon Ultra centrifugal filter unit (Merck Millipore) with a 10 kDa MWCO, and the buffer was exchanged to PBS (pH 7.4). Finally, ~6 mL of each solution was filtered through a 0.2 µm porous filter and stored at 4°C.

### GOx purification

Purification and extraction of GOx from the protein mixture were performed using the AKTA start setup (GE Healthcare) at 5°C. A HiPrep 16/60 Sephacryl S-300 high-resolution column (GE Healthcare) was used for size-exclusion protein purification. 5.5 mL of the protein mixtures were loaded onto the column and eluted using a 10 mM PBS at pH 7.4 with 140 mM NaCl. The protein presence in each collection tube was confirmed using the colorimetric GOx enzymatic activity assay, and GOx-containing fractions were collected and concentrated to 1-1.5 mL in a 10 kDa MWCO Amicon Ultra centrifugal unit. During concentration, the buffer was changed with 10 mM PBS (pH 7.0) with 10 mM EDTA (Sigma) and 150 mM NaCl. The concentration of GOx in the stock solutions were measured in a NanoDrop 2000 (flavin extinction coefficient at 450 nm is 1.4 × 10⁴ M⁻¹ cm⁻¹) (Bateman, R. C., et al. Purification and Properties of the Glucose Oxidase from Aspergillus niger. J. Biol. Chem. 240, 2209-2215 (1965)), and adjusted to 3 mg mL⁻¹ (molecular weight of GOx was determined in SDS-PAGE analysis to be -85 kDa). The final protein yields ranged between 8 to 15 mg L⁻¹ of cell culture media. GOx solutions were stored at 4°C in PBS (pH 7.0) with the addition of EDTA as an antimicrobial agent.

### SDS-PAGE analysis

1 µL of each protein solution was diluted in 9 µL of PBS (pH 7.4) and combined solution was added to 10 µL of a 2x BlueJuice loading buffer (invitrogen) containing SDS and 20 mM 1,4-dithiothreitol (DTT, Carl Roth GmbH). A commercial wild type GOx solution was prepared in PBS (pH 7.4) commercially available GOx from *A. niger* (Type II, 19 440 U g⁻¹, Sigma Aldrich). The solution was also mixed with the loading buffer. The proteins were heated to 95°C for 6 minutes while shaking at 500 rpm in 1.5 mL tubes. After, 15 µL of each solution was loaded in a gel. Gel electrophoresis was performed in a Mini-PROTEAN Tetra cell system (Bio-Rad Laboratories) at 100 V for 10 minutes and 250 V for 50 minutes. The gel was stained with a 0.25% Coomassie brilliant blue R-250 (ITW Reagents) solution in 40% ethanol and 10% acetic acid for 2 h at room temperature. The gel was then distained for 4 h in ethanol:acetic acid:water solution prepared in a 4:1:5 ratio. The gel was imaged in a Fusion Solo S gel imager (Vilber Loumat).

### Circular Dichroism (CD) spectroscopy

UV-CD spectra of GOx was measured between 195 and 250 nm using a J-810 CD spectropolarimeter (Jasco). A reference cuvette was filled with PBS (pH 7.0) with EDTA. Spectra were smoothed using a convolution filter kernel from "convolve" function. The spectra were analysed using BeStSel software to identify differences in the folding of the proteins (Micsonai, A., et al. Accurate secondary structure prediction and fold recognition for circular dichroism spectroscopy. Proceedings of the National Academy of Sciences. 112, E3095-E3103 (2015); Micsonai, A., et al. BeStSel: A web server for accurate protein secondary structure prediction and fold recognition from the circular dichroism spectra. Nucleic Acids Research. 46, W315-W322 (2018)).

### Thiol reactivity assay

The wild type and mutant GOx were reduced by 10 mM tris(2-carboxyethyl)phosphine (TCEP, abcr) for 1 hour at 5°C while shaking at 500 rpm. The TCEP was removed using a PD midiTrap G-25 desalting column (GE Healthcare) and eluted with PBS (pH 7.0) with EDTA to minimize the formation of GOx oligomers. The concentrations of GOx were adjusted to 0.87±0.04 mg mL⁻¹ using a NanoDrop 2000 spectrometer. 195 µL from each protein solution was transferred in a 96-well plate and absorption spectra were measured in the plate reader. Next, 5 µL of freshly prepared 20 mM (SPDP) (abcam) in dimethyl sulfoxide (DMSO, Sigma) was mixed with the solutions. The absorbance spectra were repeatedly measured 1, 8, 30, 60, and 120 minutes after addition of SPDP. Difference between the final absorbance and absorbance before addition of SPDP at 343 nm was compared between the proteins and a control in the absence of proteins. The number of moles of SPDP per mole of GOx was calculated according to the equation, (ΔA × MW)/(c_{GOx} × ε_{343 nm}). Where ΔA is absorbance difference at 343 nm, MW is molecular weight of GOx, c_{GOx} is concentration of GOx in mg mL⁻¹, and an extinction coefficient for pyridine-2-thione at 343 nm is ε_{343 nm} = 8.08 × 10³ M⁻¹ cm⁻¹ (Hermanson, G. T. Bioconjugate techniques 3rd edition. Elsevier Inc. (2013)).

### GOx crosslinking with PM

The GOx D70C stock solution was reduced with TCEP (as described before) and removed using the desalting column. Next, 5 µL of freshly prepared 30 mM PM in DMSO (abcr) was added to 995 µL of the freshly reduced protein solution (~1 mg mL⁻¹), PM was added proximately in 10 times excess. The reaction was performed at pH 7.0 that makes maleimide predominantly reacting to free thiols (Mukhortava, A., et al. Efficient Formation of Site-Specific Protein-DNA Hybrids Using Copper-Free Click Chemistry. Bioconjugate Chemistry. 27, 1559-1563 (2016)). The sample was incubated overnight at 4°C and shaken at 500 rpm. The free PM was removed from the PM-GOx solution using a PD midiTrap G-25 desalting column with PBS (pH 7.4) (gibco, Life Technologies). The samples were stored at 4°C.

### Fluorescence spectrometry

GOx and GOx-PM fluorescence spectra were measured in a Varioskan LUX plate reader (ThermoFisher Scientific) in bottom-scanning plate reading mode. The samples were excited at 345±2.5 nm.

### Preparation of GOx-PM-SWCNTs

50 mg of SWCNTs (CoMoCAT^{®} (7,6) enriched carbon nanotubes, Sigma Aldrich) were mixed with 50 mL of 2 wt% sodium cholate (SC) (Sigma Aldrich) and sonicated for 60 minutes using a tip sonicator (1/4 in. tip, QSonica Q700) at 1% amplitude in an ice bath. The SC-SWCNT suspension was ultracentrifuged at 164 000 × g for 3 h (Beckman Optima XPN-80). Approximately 80% of the supernatant was collected and stored at room temperature. 0.5 mL of the 25 mg L⁻¹ SC-SWCNT stock solution was mixed with 0.5 mL of ~3 mg mL⁻¹ GOx-PM (or GOx). The suspension was subsequently dialysed at 5°C in a 14 kDa MWCO dialysis tube (D9777, Sigma Aldrich) in 2 L of PBS (pH 7.4) for 4 h. The dialyzed mixture was transferred to a 300 kDa MWCO dialysis device (Spectra/Por Float-A-Lyzer, Spectrum Laboratories) and dialysed against 2 L PBS buffer for two days (during which, the buffer was replaced four times) at 5°C. The GOx-PM-SWCNTs were stored at 4°C.

### GOx removal in dialysis

Solutions of GOx in PBS were prepared using a commercial enzyme from *A. niger* (Type II, 19 440 U g⁻¹, Sigma Aldrich) and spectroscopically measured protein concentrations 0.69, 1.9, and 5.6 mg mL⁻¹ (ε_{450 nm} = 1.4 × 10⁴ M⁻¹ cm⁻¹, GOx molar weight 80 kDa) (Bateman, R., et al. Using the Glucose Oxidase/Peroxidase System in Enzyme Kinetics. Journal of Chemical Education. 12, A240-A241 (1995)) Next, 0.5 mL the solutions were mixed with a SC-SWCNT (25 mg L⁻¹) in the 1:1 ratio, and in this way the samples were diluted by half. The mixtures were dialysed in separate beakers in 300 kDa MWCO dialysis tubes (Spectrum Laboratories) against 2 L of PBS and the buffer in beakers were changed every 4 h. Fresh dialysis tubes were used on the second day of dialysis because in some of the samples were seen aggregation of SWCNT on the membranes after 24 h of the dialysis. Colorimetric ABTS GOx activity assay was performed after 24 and 48 h of dialysis according to the procedure which described above (15 µL from each suspension was used for the assay). A control dialysis experiment was performed with 1 mL of 12 mg L⁻¹ SC-SWCNTs, as well as the GOx-SWCNT enzymatic activity was compared to the GOx-PM-SWCNT sample.

### Absorbance spectroscopy

Spectra were acquired between 200 and 1350 nm using a UV-vis-NIR spectrophotometer (UV-3600 Plus, SHIMADZU). All measurements were performed in a quartz cuvette (10 mm, Quartz SUPRASIL, Hellma Analytics). SWCNT concentration was calculated using the extinction coefficient at 739 nm (ε₇₃₉ₙₘ = 25.3 mL mg⁻¹ cm⁻¹) (Yang, J., et al. Quantitative analysis of the (n,m) abundance of single-walled carbon nanotubes dispersed in ionic liquids by optical absorption spectra. Materials Chemistry and Physics. 139, 233-240 (2013)).

### NIR fluorescence spectroscopy

A 96-well plate (costar 3590, Croning Incorporated) was filed 49 µL of GOx-PM-SWCNT and GOx-SWCNT suspensions in PBS. NIR fluorescence spectra were taken between 950 and 1400 nm (75 l mm⁻¹ grating) using the NIR micro-spectrometer described previously6. The samples were illuminated at 660±5 nm laser (SuperK Extreme EXR-15 and SuperK Varia, NKT Photonics). Fluorescence spectra were continuously acquired while 1 µL of 1 M glucose in PBS (pH 7.4) was added to the well. The fluorescence of the GOx-PM-SWCNT sensor in response to the addition and removal of a 20 mM glucose solution in PBS was performed in a glass-bottom device with a 14 kDa MWCO cellulose membrane (Sigma Aldrich) on top, as described in 6.

### Example 1: Mutagenesis of GOx

GOx variants with cysteine residues, available for a crosslinker attachment to specific surface areas were designed.

To identify non-conserved surface exposed amino acids, GOx homologues were analysed and sequence conservation was mapped to the crystal structure of GOx from *Aspergillus niger* (PDB: 3QVP (Kommoju, P., et al. Probing oxygen activation sites in two flavoprotein oxidases. Biochemistry 50, 5521-5534 (2011))).

From all residues amenable for mutagenesis to a cysteine, four positions (D70, H446, A418, and K13) at different distances from the glucose-binding cavity of the enzyme were selected. Figure 1 shows a structure representation showing amino acid residues K13, D70, A418, and H446 (blue) and the internal glucose-binding cavity with FAD cofactor (red). The illustration shows distances between the glucose-binding cavity above si-face of the flavin ring (tricyclic isoalloxazine) (Kommoju, P.-R., et al. Probing oxygen activation sites in two flavoprotein oxidases. Biochemistry. 50, 5521-5534 (2011)), which is illustrated as a red sphere, and residues mutated to cysteine. FAD and glucose capturing amino acids (His559 and His516) are shown in red. The crystal structure was obtained from PDB code 3QVP.

Without wishing to be bound by theory, mutation points were selected to give the shortest distance between the active site to the sensor surface to promote the direct electron transfer from the active site of GOx to the substrate.

### Example 2: GOx expression

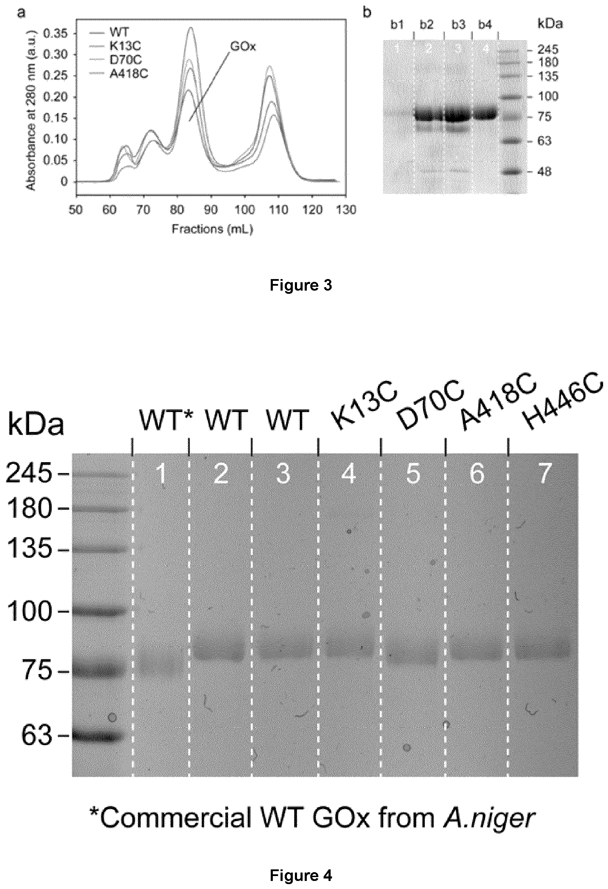

Wild type GOx and K13C, D70C, A418C, H446C variants were expressed in an optimized *P*. *pastoris* production yeast strain (Looser, V., et al. Cultivation strategies to enhance productivity of Pichia pastoris: A review. Biotechnology Advances. 33, 1177-1193 (2014)), which contains a protein N-glycosylation pathway that is similar to that found in human cells (Brooks, S. A. Appropriate glycosylation of recombinant proteins for human use: Implications of choice of expression system. Molecular Biotechnology. 28, 241-256 (2004)). The proteins were purified using size-exclusion chromatography and stored in PBS (pH 7.0) with addition of EDTA. The SDS-PAGE results shown in Figure 4 confirm an expected protein size of (~85 kDa). The recombinant GOx from *P. pastoris* show a slight increase in the apparent molecular compared to the commercial GOx from *A. niger* (~80 kDa) (lane 1). This difference is attributed to the greater degree of glycosylation.

The expressed GOx variants were concentrated and purified by size-exclusion chromatography and were achieving yields of 8 to 15 mg L⁻¹ of a cell culture media. The results of the chromatograph are shown in Figure 3.

GOx solutions were stored in sodium phosphate buffer saline (PBS) at pH 7.0 with addition of an antimicrobial agent (ethylenediaminetetraacetic acid, EDTA) (Finnegan, S. et al. EDTA: An Antimicrobial and Antibiofilm Agent for Use in Wound Care. Advances in Wound Care. 4, 415-412 (2015)).

### Example 3: Characterisation of wild type GOx and the mutants K13C, D70C, A418C, and H446C

### SDS-PAGE analysis

The proteins were analysed by a sodium dodecyl sulfate polyacrylamide gel electrophoresis (SDS-PAGE) shown in Figure 4. The SDS-PAGE revealed an increase in the apparent molecular weight of *P. pastoris*-expressed GOx is -85 kDa compared to the commercial GOx from *A. niger* (lane 1) which is likely due to the greater degree of glycosylation. GOx from *A. niger* is 10-16% glycosylated and has a molecular weight of -80 kDa. See reference 28. Among the variants the D70C (lane 5) has a slightly smaller molecular weight.

### Circular dichroism (CD) spectroscopy

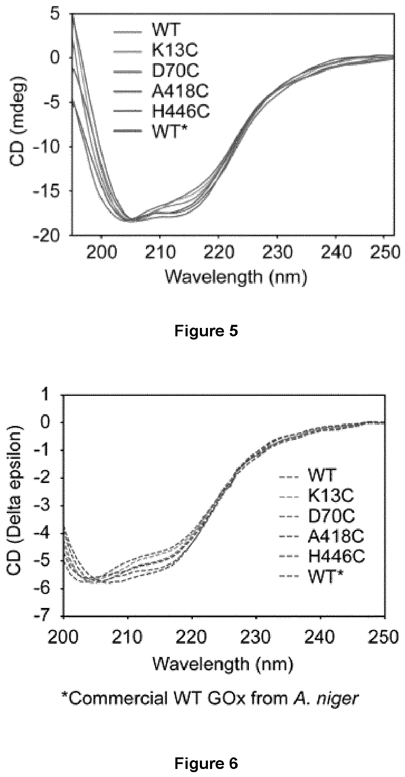

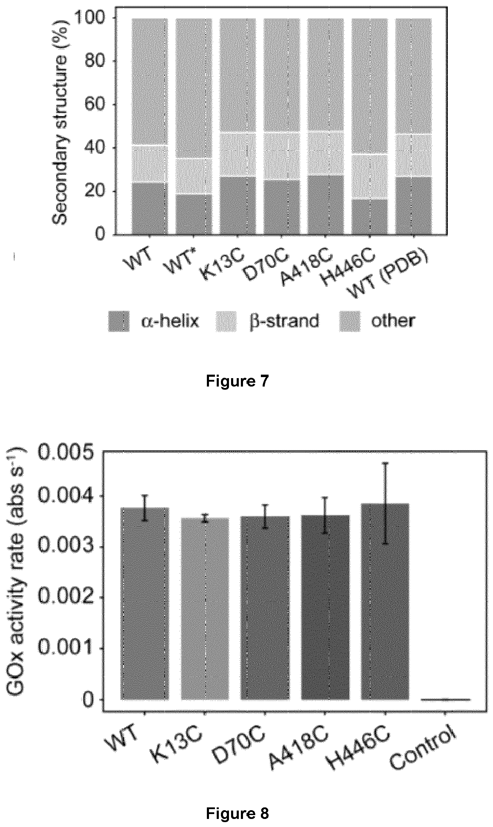

The secondary structure of the enzymes was characterised with the circular dichroism (CD) measurements shown in Figure 5, and the fitted theoretical spectra shown in Figure 6. All structures, including the expressed wild type, commercial wild type, and mutants, share similar CD spectra. The CD spectra of GOx variants was fitted using a secondary structure prediction software BeStSel. The results are shown in Figures 6 and 7. Figure 6 shows the fitted CD spectra of GOx variants using a secondary structure prediction software BeStSel. Figure 7 shows the distribution of BeStSel-predicted percentage of structure features (a-helixes and β-strands) in the GOx variants. The variants are compared to the cristal structure from the PDB 1CF3 code. A star symbol indicates the sample which was made of commercial GOx obtained from A. *niger.* The expressed proteins contain 17 - 28% α-helixes and 16 - 22% β-strands, Figure 7, in accordance with values reported for the commercial GOx (24 and 17% for α-helixes and β-strands, respectively). See refreence15. The results confirmed that the mutations did not significantly diminish protein folding.

### Enzymatic activity in ABTS assay

The preservation of enzymatic activity was also confirmed by comparing activities measured with the colorimetric ABTS assay (Figure 8). As shown in the figure, the enzymatic activity towards glucose does not differ between wild type GOx and the mutants, including that the mutations had no significant effect on activity.

### Example 4: Bioconjugation of GOx with thiol reactive crosslinkers

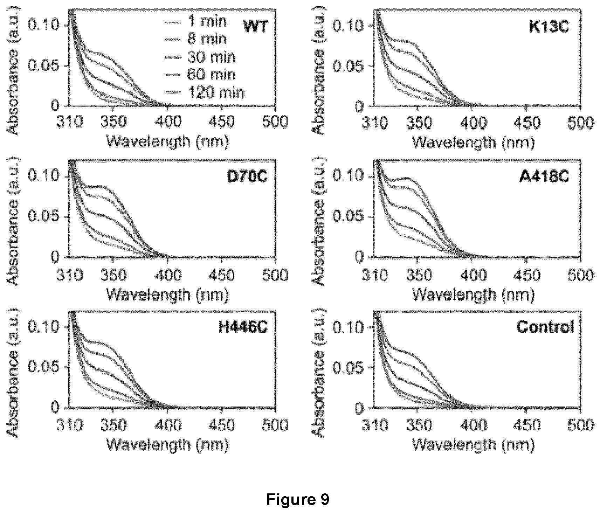

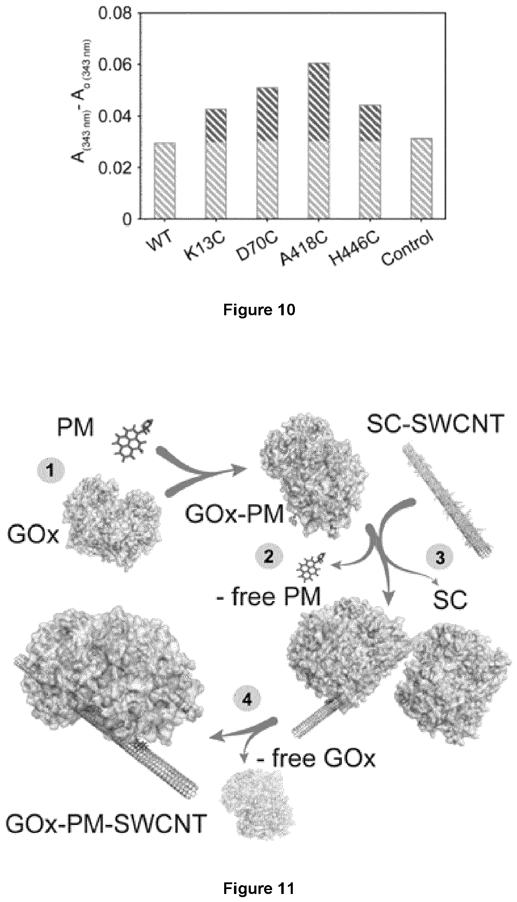

The accessibility of the targeted residues were compared based on their reactivity towards succinimidyl 3-(2-pyridyldithio)propionate (SPDP), which releases conjugation reaction product, pyridine-2-thione, that absorbs at 343 nm30. Absorption spectProteins were prepared for crosslinking by reducing thiols using tris(2-cra were measured over 120 minutes (Figure 9) and the reactivity of free thiols was monitored through the increase of absorption at 343 nm (Figure 10). It can be seen that all mutated GOx demonstrated increased absorbance at 343 nm indicating the presence of accessible surface thiols in the mutated proteins. In contrast, WT GOx (which was also reduced) did not react with SPDP since its absorbance increase at 343 nm was similar to the control (SPDP spontaneously degrades in aqueous solutions) (Kavimandan, N., et al. Synthesis and Characterization of Insulin-Transferrin Conjugates. Biocojugate Chemistry. 17, 1376-1384 (2006)).

A418C was identified as the most reactive cysteine mutant, with -0.4 moles of SPDP reacted per mole of GOx, followed by the D70C (-0.3 moles of SPDP reacted per mole of GOx). As expected, the reduced wild type GOx did not react with SPDP.

The D70C variant was selected to demonstrate a thiol-maleimide "click" chemistry with a PM crosslinker to immobilise the proteins onto SWCNTs.

### Example 5: Protein immobilisation onto SWCNTs

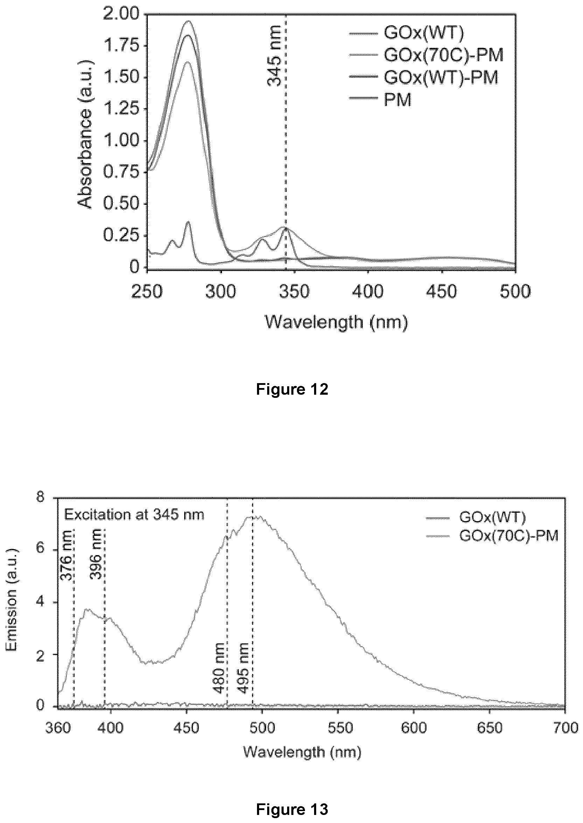

The D70C variant was selected for immobilisation onto the SWCNT using the conjugation strategy shown in Figure 11. The absorbance measurements shown in Figure 12 confirm the presence of PM in the purified conjugated protein fractions. The conjugation of the PM linker to the GOx is also confirmed by the PM fluorescence measurements shown in Figure 13. In agreement with previous observations, the PM linker emits fluorescence at 376 and 396 nm upon 345 nm excitation when a covalent bond is formed between the PM maleimide and the protein thiol (Wu, C., et al. N-(1-Pyrene)maleimide: A Fluorescent Cross-Linking Reagent. Biochemistry. 15, 2863-2868 (1976)). Broad emission peaks at 480 and 495 nm also appear due to the proximity of the two pyrene to one another (~10 Å) (Bains, G. et al. Pyrene: A probe to study protein conformation and conformational changes. Molecules. 16, 7909-7935 (2011)), indicating that PM-GOx monomers were folded into enzymatically active dimers.

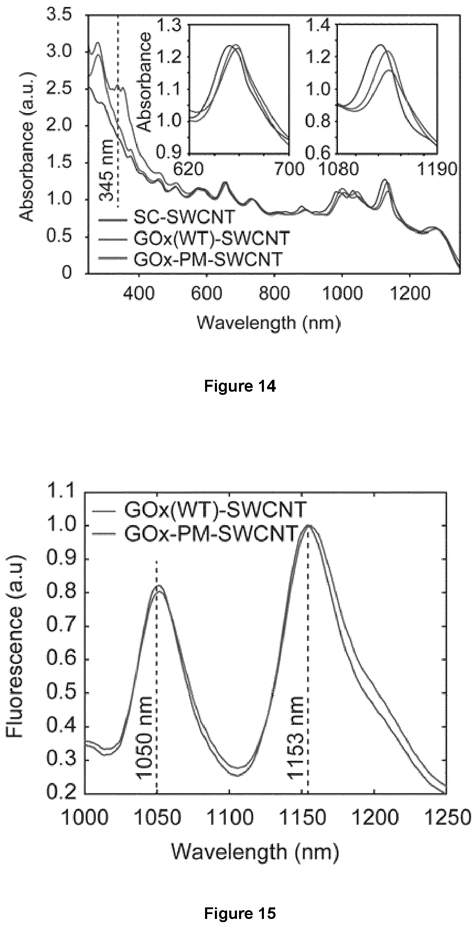

Protein conjugation to the SWCNT surface was confirmed through both absorbance and NIR fluorescence spectroscopy (Figures 14, and 15). Compared to SC-SWCNTs, both non-specifically adsorbed GOx(WT)-SWCNTs and GOx-PM-SWCNTs show red-shifted absorption peaks for the (7,5) and (7,6) chiralities of SWCNTs. The merged peak of the *E₂₂* (7,5) and *E₂₂* (7,6) SWCNTs was shifted from 657 to 660 nm. The *E₁₁* (7,5) and *E₁₁* (7,6) SWCNT peaks shifted from 1045 to 1047 nm and from 1135 to 1138 nm, respectively. This is attributed to the change in solvation due to the protein corona. In contrast to the GOx(WT)-SWCNTs, the GOx-PM-SWCNT sample shows additional absorption peaks that correspond to absorption of the PM linker. A comparison of the SWCNT fluorescence shown in Figure 15 similarly shows distinct spectra for the GOx(WT)-SWCNT and GOx-PM-SWCNT samples. The *E₁₁* emissions of (7,5) and (7,6) chiralities were observed at 1050±1 nm and 1153±1 nm, respectively, for GOx-PM-SWCNTs on 660 nm excitation. These emission peaks were red-shifted 2±1 nm and 4±1 nm for the (7,5) and (7,6) chiralities, respectively, for the GOx(WT)-SWCNTs.

This red-shifting suggests increased surface coverage of GOx-PM-SWCNTs compared to the GOx-SWCNTs (Choi, J., et al. Solvatochromism in single-walled carbon nanotubes. Applied Physics Letters. 90, 1-3 (2007); Jeng, E., et al. Detection of DNA hybridization using the near-infrared band-gap fluorescence of single-walled carbon nanotubes. Nano Letters. 6, 371-375 (2006)).

As such, it has been shown that the thiol linked modified biomolecules according to the present invention can cover the SWCNT surface more densely than prior techniques. Achieving tighter SWCNT surface coverage for the GOx-PM-SWCNT suspension may positively impact sensor stability and/or analyte sensitivity. Either effect is valuable for a sensor.

The present invention enables the orientation of proteins on the SWCNT surface. The present invention also significantly improves the SWCNT surface coverage with the proteins adhered through the linkers of the present invention, for example the PM linker. Higher protein coverage density enhances the optical response of the sensor to the analyte. The present method also has a further significant advantage over other approaches. The protein immobilisation method reported in this work site-specifically cross-links the biomolecule with the linker (for example PM) and then, secondly immobilises it on the substrate (for example SWCNT). This has advantages over, for example an approach where DNA is wrapped around the SWCNT prior to linking a protein with suitable functional groups to the ends of the DNA molecule, for example by "click" chemistry. The "click" chemistry reactions are known to have yields less that 70-90% in solutions when the reagents are mobile (Kim, Y., et al. Efficient site-specific labeling of proteins via cysteines. Bioconjugate Chemistry. 19, 786-791 (2008)). The reaction yields could be significantly reduced when the DNAs are adsorbed on the substrate surface. The advantage of the method described in this invention is that the conjugation occurs in the solution and the reaction product can be purified before its site-specific immobilisation onto the substrate.

### Example 6: Amount of GOx immobilised onto SWCNTs

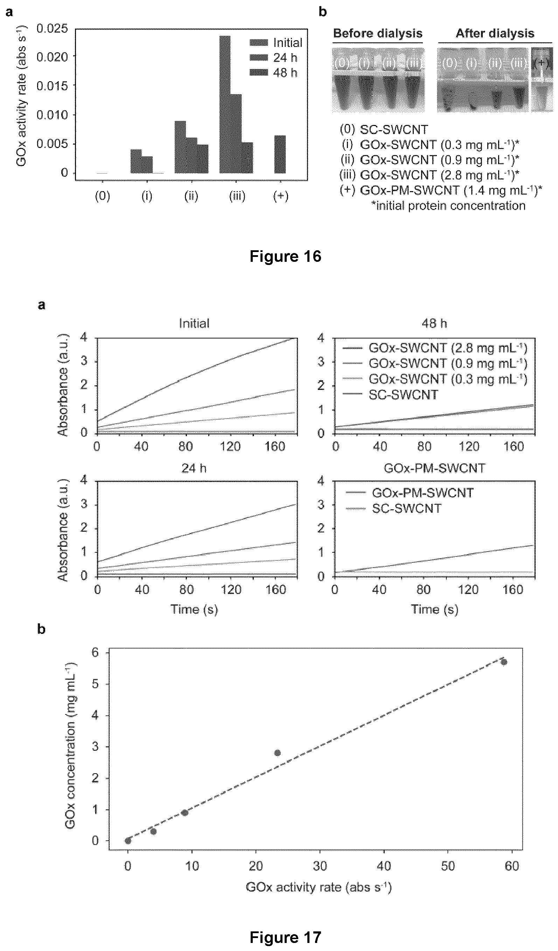

The effect of GOx concentration on SWCNT suspension was explored. Solutions of GOx were prepared in PBS and protein concentrations were spectroscopically measured. Next, the solutions were mixed with SC-SWCNTs (25 mg L⁻¹) in a ratio 1 to 1, and obtained (i) 0.3, (ii) 0.9, and (iii) 2.8 mg mL⁻¹ final concentrations of GOx. The mixtures were dialysed exchanging the buffer every 4 h. The colorimetric GOx activity assay was performed after 24 and 48 h of dialysis and the results are shown in Figure 16a. The enzymatic activity rates in the suspension were gradually reduced during dialysis dominantly due to removal of free GOx from the suspensions. The activity of the protein was linearly proportional to GOx concentration in the solutions (Figure 17). Complete aggregation of SWCNTs was observed for the (0) SC-SWCNT sample when the surfactant was dialysed. The GOx activity in samples (ii) and (iii) levelled at -0.005 abs s⁻¹ after 48 h of dialysis, hence, only the SWCNT surface-adsorbed GOx was retained in the suspensions. SWCNTs in the sample (i) aggregated after two days of the dialysis, we assume that 0.3 mg mL⁻¹ of GOx was not sufficient to completely cover the SWCNT surface. *Karajanagi et al.* observed that protein-SWCNTs suspensions aggregate upon removal of excess proteins via dialysis since the adsorbed proteins are in equilibrium with solution-phase proteins (Karajanagi, S. S., et al. Protein-assisted solubilization of single-walled carbon nanotubes. Langmuir. 22, 1392-1395 (2006)). However, our results show that the aggregation of SWCNTs depends on the ratio between initial concentrations of GOx and SWCNTs. Based on the experimental results, for the GOx-PM-SWCNT wrapping we have chosen approximately 1:120 SWCNT to GOx mass ratio, that was used in the SWCNT surface activation method.

### Example 7: Glucose detection using the GOx-PM-SWCNT sensor

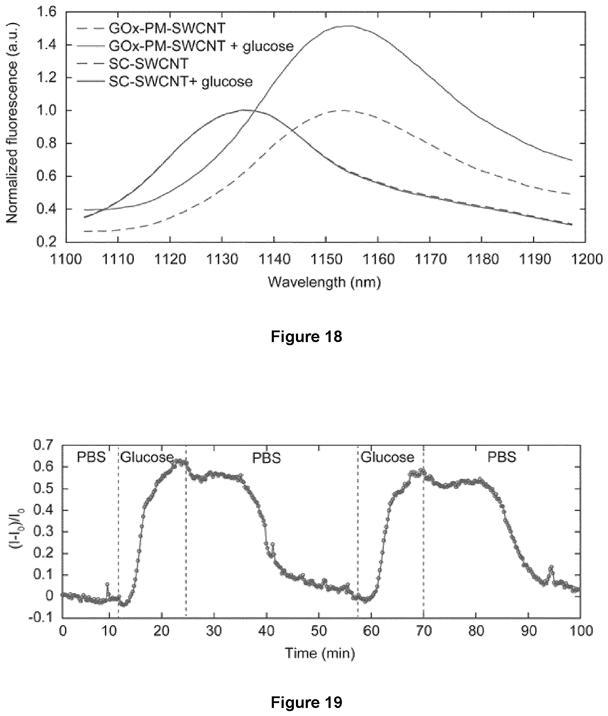

The performance of the GOx-PM-SWCNT sensors was evaluated by monitoring the fluorescence intensity peak at 1153±1 nm upon glucose addition (Figure 18). In the presence of 20 mM glucose, was observed an increase in fluorescence intensity. On the other hand, no fluorescence response was observed for the SC-SWCNTs. We explored the reversibility of this response using a device with a permeable membrane, as described in our previous work, reference 6 (Figure 19). A 20 mM glucose solution was added to the top of the device to trigger a response, and the device was washed with PBS after the signal reached a plateau to revert the response. Approximately 10 minutes after addition of glucose, the fluorescence intensity increased by 60%, and buffer washing completely diminished the fluorescence back to its initial intensity.

The sensors according to the present invention can therefore be used as the sensing element in continuous glucose monitoring devices, for example implantable continuous glucose monitoring devices. This is particularly true since the sensor does not contain any toxic reaction mediators which were present in earlier designs of SWCNT-based sensors (Barone, P., et al. Near-infrared optical sensors based on single-walled carbon nanotubes. Nature Materials. 4, 86-92 (2004)).

In conclusion, the present invention provides sensors which offer advantages over sensors current known in the art. The present invention provides sensors which allow for the orientation of the biomolecule on the substrate to be optimised. Such optimisation includes improving protein folding by immobilising the protein on the substrate via a linker at a defined site on the protein. Such optimisation includes orienting the biomolecule on the substrate to maximise performance, for example improving charge transfer to the substrate. Such optimisation includes improving sensor stability by providing covalent links to the biomolecule and strong links between the linker and substrate. The present invention also enables the surfactant-exchange-based sensor preparation method which is scalable and, therefore, attractive for commercial production.

In particular, biosensors, for example optical sensors or electrochecmical sensors have been developed using a modified enzyme linked *via* a linker to a SWCNTs. For example, an optical glucose sensor has been developed using genetically engineered GOx. The cysteine-containing mutants were specifically linked to SWCNTs using a PM linker. Thus, the present invention provides for modified enzymes immobilised in a site-specific way which offers performance advantages. To achieve this, for example, variants with single-cysteine mutations on the surface of the enzyme were developed which were shown to retain enzyme activity but direct site specific linkage to a linker molecule and thereafter immobilisation to the SWCNT. Successful immobilisation of PM-GOx onto SWCNTs was confirmed in absorbance and NIR fluorescence measurements. The blueshifts of the SWCNT peaks in the NIR fluorescence spectrum of GOx-PM-SWCNTs in comparison to GOx-SWCNTs indicated that GOx-PM has denser coverage of the SWCNT surface than non-specifically adsorbed GOx. The sensor shows a reversible response to a change of glucose, which can be applicable for continuous glucose monitoring.

However, it should be understood that other enzymes can also be used to develop sensors for other analytes. For example, cholesterol oxidase or lactate oxidase can be used to form sensors for cholesterol and lactate detection.

No doubt many other effective alternatives will occur to the skilled person. It will be understood that the invention is not limited to the described embodiments and encompasses modifications apparent to those skilled in the art and lying within the spirit and scope of the claims appended hereto.

The invention will now be described in relation to the following clauses:
1. A sensor B-L-S, wherein B is a biomolecule comprising one or more surface-exposed thiol moieties, L is a linker comprising a thiol reactive group, and S is a substrate; wherein L is covalently linked to B *via* the one or more surface-exposed thiol moieties.
2. A sensor comprising a biomolecule immobilised to a substrate; wherein the biomolecule is immobilised to the substrate *via* a linker; and wherein the biomolecule has one or more surface accessible thiol moieties and is connected to the linker *via* said one or more surface accessible thiol moieties.
3. A sensor comprising a mutated biomolecule immobilised to a substrate; wherein the biomolecule is immobilised to the substrate *via* a linker; wherein the biomolecule is mutated to introduce one or more surface accessible thiol moieties; and wherein the biomolecule is connected to the linker *via* said one or more surface accessible thiol moieties.
4. A sensor comprising a biomolecule immobilised to a substrate *via* a linker, wherein the linker is connected to the biomolecule through a thiol linkage, and wherein the substrate is a material having optical or charge carrying properties.
5. The sensor of clause 4, wherein the thiol linkage is between one or more surface accessible thiol moieties on the biomolecule and thiol reactive group(s) on the linker.
6. A sensor comprising a biomolecule immobilised to a carbon nanomaterial substrate *via* a linker, wherein the linker is connected to the biomolecule through a thiol linkage.
7. The sensor according to any preceding clause, wherein the biomolecule is glucose oxidase, concanavalin A, glucose binding protein, cholesterol oxidase or lactate oxidase.
8. The sensor according to any preceding clause, wherein the biomolecule comprises one or more mutations, wherein said one or more mutations introduce said one or more surface-exposed thiol moieties.
9. The sensor according to clause 5 wherein said mutation is a substitution, wherein preferable the substituted residue is selected from a cysteine, selenocysteine or a non-natural amino acid including a thiol group.
10. The sensor according to any preceding clause, wherein the substrate is a carbon nanomaterial, preferably a carbon nanocarrier, single walled carbon nanotube (SWCNT), graphene, fullerene, multi-walled carbon nanotube (MWCNT) or single-walled carbon nanohorn, more preferably a single walled carbon nanotube (SWCNT).
11. The sensor according to any preceding clause, wherein the linker comprises a thiol reactive group, preferably wherein the thiol reactive group is selected from maleimides, pyridyl dusulfides, aziridines, acryloyl derivatives, arylating agents, disulfide reagents, vinylsulfone derivatives, haloacetyls including iodoacetyl and bromoacetyl, more preferably wherein the thiol reactive group is a maleimide.
12. The sensor according to any preceding clause, wherein the linker comprises a substrate adherence group, preferably wherein the substrate adherence group non-covalently adheres to the surface of the substrate, more preferably wherein the substrate adherence groups is selected form pyrenyl, porphyrin, triphenylene, anthracene, dibenzocyclooctyne, pentacene.
13. The sensor according to any preceding clause, wherein the substrate comprises a plurality of biomolecules immobilised on its surface, and wherein the biomolecules are immobilised in an ordered manner.
14. A method of controlling biomolecules immobilised on a carbon nanomaterial substrate, said method comprising: modifying said biomolecules to create biomolecules having a surface accessible thiol moieties, and immobilising said modifying biomolecules to said substrate using linkers having thiol reactive groups, wherein the biomolecules are connected to the linkers via the surface accessible thiol moieties and the thiol reactive groups.
15. A method of attaching a biomolecule to a carbon nanomaterial substrate comprising: modifying the biomolecule to introduce one or more surface-exposed thiol moieties; attaching a linker to the biomolecule wherein the linker comprises a thiol reactive group and a substrate adherence group; wherein the linker attaches to the biomolecule *via* the thiol group and thiol reactive group; and wherein the biomolecule-linker adheres to the substrate *via* the substrate adherence group.
16. A method of obtaining structured immobilisation of biomolecules on a substrate comprising modifying the biomolecules to comprise one or more surface-exposed thiol moieties; attaching linking groups to the biomolecules *via* the surface-exposed thiol moieties and a thiol reactive group on the linking group; and adhereing the biomolecules to the substrate *via* the linking groups.
17. The method of clauses 14 to 16, wherein the biomolecule has one surface accessible thiol moiety.
18. A glucose oxidase polypeptide comprising one or more mutations, a carbohydrate oxidase polypeptide comprising one or more mutations or a lactate oxidase polypeptide comprising one or more mutations; wherein said one or more mutations introduces a site-selective thiol conjugation site selected to permit conjugation of the polypeptide to a linker comprising a thiol reactive group.
19. The polypeptide of clause 18, wherein the mutation is a substitution, wherein preferably the substituted residue is selected from a cysteine or a non-natural amino acid containing a thiol group.
20. The polypeptide of clause 19, wherein the substituted residue is cysteine.
21. The polypeptide of any one of clauses 18 to 20, wherein the sequence of glucose oxidase comprises the sequence defined in SEQ ID NO: 1 or a functional variant thereof and wherein the sequence further comprises a mutation at one or more of positions 13, 70, 418 and 446 in SEQ ID NO: 1.
22. The glucose oxidase polypeptide of clause 21, wherein the sequence of glucose oxidase is selected from SEQ ID NO: 2, 3, 4 or 5 or a functional variant thereof, wherein X is selected from a cysteine or a non-natural amino acid containing a thiol group.
23. A polynucleotide encoding the polypeptides of any of clauses 18 to 22.
24. A nucleic acid construct comprising the polynucleotides of clause 23 operably linked to a regulatory sequence.
25. A method of producing a sensor, comprising:
   a) providing a biomolecule comprising one or more surface exposed thiol moieties;
   b) contacting the biomolecule with a linker comprising a thiol reactive group such that the linker covalently links to the biomolecule *via* the thiol reactive group;
   c) adhering the biomolecule-linker to a substrate, wherein the biomolecule-linker adheres *via* the linker.
26. Use of a sensor according to the present invention in the detection and/or quantification of an analyte in a sample.
27. A method of detecting and/or quantifying an analyte in a sample comprising the use of a sensor according to the present invention.

### References:

1. Kim, J., Campbell, A. S. & Wang, J. Wearable non-invasive epidermal glucose sensors: A review. Talanta 177, 163-170 (2018).
2. Chen, C. et al. Current and emerging technology for continuous glucose monitoring. Sensors (Switzerland) 17, 1-19 (2017).
3. Kropff, J. et al. Accuracy and longevity of an implantable continuous glucose sensor in the PRECISE study: A 180-day, prospective, multicenter, pivotal trial. Diabetes Care 40, 63-68 (2017).
4. Lundsgaard-Nielsen, S. M. et al. Critical-depth Raman spectroscopy enables home use non-invasive glucose monitoring. PLoS One 13, 1-11 (2018).
5. Feng, W. & Ji, P. Enzymes immobilized on carbon nanotubes. Biotechnol. Adv. 29, 889-895 (2011).
6. Zubkovs, V., Schuergers, N., Lambert, B., Ahunbay, E. & Boghossian, A. A.Mediatorless, Reversible Optical Nanosensor Enabled through Enzymatic PocketDoping. Small 1701654, 1-10 (2017).
7. Yoon, H. et al. Periplasmic binding proteins as optical modulators of single-walled carbon nanotube fluorescence: Amplifying a nanoscale actuator. Angew. Chemie - Int. Ed. 50, 1828-1831 (2011).
8. Oliveira, S. F. et al. Protein functionalized carbon nanomaterials for biomedical applications. Carbon N. Y. 95, 767-779 (2015).
9. Muguruma, H., Yoshida, S., Urata, M., Fujisawa, K. & Matsui, Y. An Amperometric Biosensor for Glucose Based on a Composite Electrode of Glucose Dehydrogenase, Carbon Nanotubes, and Plasma-polymerized Thin Films. Electrochemistry 76, 545-548 (2008).
10. Ferri, S., Kojima, K. & Sode, K. Review of Glucose Oxidases and Glucose Dehydrogenases: A Bird's Eye View of Glucose Sensing Enzymes. J. Diabetes Sci. Technol. 5, 1068-1076 (2011).
11. Sanz, V., Coley, H. M., Silva, S. R. P. & McFadden, J. Modeling the binding of peptides on carbon nanotubes and their use as protein and DNA carriers. J. Nanoparticle Res. 14, 1-13 (2012).
12. Ge, C. et al. Binding of blood proteins to carbon nanotubes reduces cytotoxicity. Proc. Natl. Acad. Sci. 108, 16968-16973 (2011).
13. Williams, R. M. et al. Noninvasive ovarian cancer biomarker detection via an optical nanosensor implant. Sci. Adv. 4, 1-11 (2018).
14. Bisker, G. et al. Protein-targeted corona phase molecular recognition. Nat. Commun. 7, 1-14 (2016).
15. Wohlfahrt, G. et al. 1.8 and 1.9 A resolution structures of the Penicillium amagasakiense and Aspergillus niger glucose oxidases as a basis for modelling substrate complexes. Acta Crystallogr. Sect. D Biol. Crystallogr. 55, 969-977 (1999).
16. Gouda, M. D., Singh, S. A., Rao, A. G. A., Thakur, M. S. & Karanth, N. G. Thermal inactivation of glucose oxidase: Mechanism and stabilization using additives. J. Biol. Chem. 278, 24324-24333 (2003).
17. Filla, N., Ramasamy, R. & Wang, X. Forces, energetics, and dynamics of conjugatedcarbon ring tethers adhered to CNTs: a computational investigation. Phys. Chem. Chem. Phys. 20, 11327-11335 (2018).
18. Yang, Z., Wang, Z., Tian, X., Xiu, P. & Zhou, R. Amino acid analogues bind to carbon nanotube via ππ - Interactions: Comparison of molecular mechanical and quantum mechanical calculations. J. Chem. Phys. 136, 1-10 (2012).
19. He, Z. & Zhou, J. Probing carbon nanotube-amino acid interactions in aqueous solution with molecular dynamics simulations. Carbon N. Y. 78, 500-509 (2014).
20. Kommoju, P., Chen, Z., Bruckner, R. C., Mathews, F. S. & Jorns, M. S. Probing oxygen activation sites in two flavoprotein oxidases. Biochemistry 50, 5521-5534 (2011).
21. Looser, V. et al. Cultivation strategies to enhance productivity of Pichia pastoris: A review. Biotechnol. Adv. 33, 1177-1193 (2014).
22. Witt, S., Singh, M. & Kalisz, H. M. Structural and kinetic properties of nonglycosylated recombinant Penicillium amagasakiense glucose oxidase expressed in Escherichia coli. Appl. Environ. Microbiol. 64, 1405-1411 (1998).
23. Brooks, S. A. Appropriate glycosylation of recombinant proteins for human use: Implications of choice of expression system. Mol. Biotechnol. 28, 241-256 (2004).
24. Hamilton, S. R. & Gerngross, T. U. Glycosylation engineering in yeast: the advent of fully humanized yeast. Curr. Opin. Biotechnol. 18, 387-392 (2007).
25. Krainer, F. W. et al. Knockout of an endogenous mannosyltransferase increases the homogeneity of glycoproteins produced in Pichia pastoris. Sci. Rep. 3, 1-13 (2013).
26. Gu, L., Zhang, J., Liu, B., Du, G. & Chen, J. High-level extracellular production of glucose oxidase by recombinant Pichia pastoris using a combined strategy. Appl. Biochem. Biotechnol. 175, 1429-47 (2015).
27. Finnegan, S. & Percival, S. L. EDTA: An Antimicrobial and Antibiofilm Agent for Use in Wound Care. Adv. Wound Care 4, 415-421 (2015).
28. Leskovac, V., Trivic, S., Wohlfahrt, G., Kandrac, J. & Pericin, D. Glucose oxidase from Aspergillus niger: The mechanism of action with molecular oxygen, quinones, and oneelectron acceptors. Int. J. Biochem. Cell Biol. 37, 731-750 (2005).
29. Bateman, R. C. & Evans, J. A. Using the Glucose Oxidase/Peroxidase System in Enzyme Kinetics. J. Chem. Educ. 72, 240-241 (1995).
30. Kavimandan, N. J., Losix, E., Wilson, J. J., Brodbelt, J. S. & Peppas, N. A. Synthesis and Characterization of Insulin-Transferrin Conjugates. Biocojugate Chem. 17, 1376- 1384 (2006).
31. Hermanson, G. T. Bioconjugate Techniques. (Elsevier Inc., 2013).
32. Wu, C. W., Yarbrough, L. R. & Wu, F. Y. H. N-(1-Pyrene)maleimide: A Fluorescent Cross-Linking Reagent. Biochemistry 15, 2863-2868 (1976).
33. Bains, G., Patel, A. B. & Narayanaswami, V. Pyrene: A probe to study protein conformation and conformational changes. Molecules 16, 7909-7935 (2011).
34. Jeng, E. S., Moll, A. E., Roy, A. C., Gastala, J. B. & Strano, M. S. Detection of DNA hybridization using the near-infrared band-gap fluorescence of single-walled carbon nanotubes. Nano Lett. 6, 371-375 (2006).
35. Choi, J. H. & Strano, M. S. Solvatochromism in single-walled carbon nanotubes. Appl. Phys. Lett. 90, 9-12 (2007).
36. Karajanagi, S. S., Vertegel, A. A., Kane, R. S. & Dordick, J. S. Structure and function of enzymes adsorbed onto single-walled carbon nanotubes. Langmuir 20, 11594- 11599 (2004).
37. Saifuddin, N., Raziah, A. Z. & Junizah, A. R. Carbon nanotubes: A review on structure and their interaction with proteins. J. Chem. 676815, 1-18 (2013).
38. Karajanagi, S. S. et al. Protein-assisted solubilization of single-walled carbon nanotubes. Langmuir 22, 1392-1395 (2006).
39. Zubkovs, V. et al. Spinning-disc confocal microscopy in the second near-infrared window (NIR-II). Sci. Rep. 8, 1-10 (2018).
40. Barone, P. W., Baik, S., Heller, D. A. & Strano, M. S. Near-infrared optical sensors based on single-walled carbon nanotubes. Nat. Mater. 4, 86-92 (2004).
41. Ashkenazy, H., Erez, E., Martz, E., Pupko, T. & Ben-Tal, N. ConSurf 2010: Calculating evolutionary conservation in sequence and structure of proteins and nucleic acids. Nucleic Acids Res. 38, 529-533 (2010).
42. Edelheit, O., Hanukoglu, A. & Hanukoglu, I. Simple and efficient site-directed mutagenesis using two single-primer reactions in parallel to generate mutants for protein structure-function studies. BMC Biotechnol. 9, 1-8 (2009).
43. Lin-Cereghino, J. et al. Condensed protocol for competent cell preparation and transformation of the methylotrophic yeast Pichia pastoris. Biotechniques 38, 44-48 (2005).
44. Swoboda, B. E. P. & Massey, V. Purification and Properties of the Glucose Oxidase from Aspergillus niger. J. Biol. Chem. 240, 2209-2215 (1965).
45. Micsonai, A. et al. BeStSel: A web server for accurate protein secondary structure prediction and fold recognition from the circular dichroism spectra. Nucleic Acids Res. 46, W315-W322 (2018).
46. Micsonai, A. et al. Accurate secondary structure prediction and fold recognition for circular dichroism spectroscopy. Proc. Natl. Acad. Sci. 112, E3095-E3103 (2015).
47. Mukhortava, A. & Schlierf, M. Efficient Formation of Site-Specific Protein-DNA Hybrids Using Copper-Free Click Chemistry. Bioconjug. Chem. 27, 1559-1563 (2016).
48. Yang, J., Zhang, Z., Zhang, D. & Li, Y. Quantitative analysis of the (n,m) abundance of single-walled carbon nanotubes dispersed in ionic liquids by optical absorption spectra. Mater. Chem. Phys. 139, 233-240 (2013).
49. Noncovalent interaction of single-walled nanotubes with 1-pyrenebutanoic acid suceimide ester and glucoseoxidase (2011) J. of Physical Chemistry C).
50. Carbon nanomaterials for biological imaging and nanomedical therapy" G. Hong, S. Diao, A. L. Antaris, H. Dai (2015) Chemical Reviews
51. Williams, R., et al. Noninvasive ovarian cancer biomarker detection via an optical nanosensor implant. Science Advances. 4, 1-11 (2018)
52. Florian, A., et al. Nanobody conjugated nanotubes for targeted near-infrared in vivo imaging and sensing. Angewandte Chemie Int. Ed. 10.1002/ange.201904167 (2019)

### SEQUENCE LISTING

SEQ ID NO: 1 (wild type GOx) 
SNGIEASLLTDPKDVSGRTVDYIIAGGGLTGLTTAARLTENPNISVLVIESGSYESDRG
PIIEDLNAYGDIFGSSVDHAYETVELATNNQTALIRSGNGLGGSTLVNGGTWTRPHKA
QVDSWETVFGNEGWNWDNVAAYSLQAERARAPNAKQIAAGHYFNASCHGVNGTV
HAGPRDTGDDYSPIVKALMSAVEDRGVPTKKDFGCGDPHGVSMFPNTLHEDQVRS
DAAREWLLPNYQRPNLQVLTGQYVGKVLLSQNGTTPRAVGVEFGTHKGNTHNVYA
KHEVLLAAGSAVSPTILEYSGIGMKSILEPLGIDTVVDLPVGLNLQDQTTATVRSRITSA
GAGQGQAAWFATFNETFGDYSEKAHELLNTKLEQWAEEAVARGGFHNTTALLIQYE
NYRDWIVNHNVAYSELFLDTAGVASFDVWDLLPFTRGYVHILDKDPYLHHFAYDPQY
FLNELDLLGQAAATQLARNISNSGAMQTYFAGETIPGDNLAYDADLSAWTEYIPYHFR
PNYHGVGTCSMMPKEMGGVVDNAARVYGVQGLRVIDGSIPPTQMSSHVMTVFYAM
ALKISDAILEDYASMQ
SEQ ID NO: 2 (mutation at position 13) 
SNGIEASLLTDPXDVSGRTVDYIIAGGGLTGLTTAARLTENPNISVLVIESGSYESDRGP
IIEDLNAYGDIFGSSVDHAYETVELATNNQTALIRSGNGLGGSTLVNGGTWTRPHKAQ
VDSWETVFGNEGWNWDNVAAYSLQAERARAPNAKQIAAGHYFNASCHGVNGTVHA
GPRDTGDDYSPIVKALMSAVEDRGVPTKKDFGCGDPHGVSMFPNTLHEDQVRSDAA
REWLLPNYQRPNLQVLTGQYVGKVLLSQNGTTPRAVGVEFGTHKGNTHNVYAKHEV
LLAAGSAVSPTILEYSGIGMKSILEPLGIDTVVDLPVGLNLQDQTTATVRSRITSAGAGQ
GQAAWFATFNETFGDYSEKAHELLNTKLEQWAEEAVARGGFHNTTALLIQYENYRD
WIVNHNVAYSELFLDTAGVASFDVWDLLPFTRGYVHILDKDPYLHHFAYDPQYFLNEL
DLLGQAAATQLARNISNSGAMQTYFAGETIPGDNLAYDADLSAWTEYIPYHFRPNYHG
VGTCSMMPKEMGGVVDNAARVYGVQGLRVIDGSIPPTQMSSHVMTVFYAMALKISD
AILEDYASMQ
 wherein X is selected from a cysteine or an amino acid including a thiol or selenol group, for example selenocysteine
SEQ ID NO: 3 (mutation at position 70) 
SNGIEASLLTDPKDVSGRTVDYIIAGGGLTGLTTAARLTENPNISVLVIESGSYESDRGP
IIEDLNAYGXIFGSSVDHAYETVELATNNQTALIRSGNGLGGSTLVNGGTWTRPHKAQ
VDSWETVFGNEGWNWDNVAAYSLQAERARAPNAKQIAAGHYFNASCHGVNGTVHA
GPRDTGDDYSPIVKALMSAVEDRGVPTKKDFGCGDPHGVSMFPNTLHEDQVRSDAA
 
REWLLPNYQRPNLQVLTGQYVGKVLLSQNGTTPRAVGVEFGTHKGNTHNVYAKHEV
LLAAGSAVSPTILEYSGIGMKSILEPLGIDTVVDLPVGLNLQDQTTATVRSRITSAGAGQ
GQAAWFATFNETFGDYSEKAHELLNTKLEQWAEEAVARGGFHNTTALLIQYENYRD
WIVNHNVAYSELFLDTAGVASFDVWDLLPFTRGYVHILDKDPYLHHFAYDPQYFLNEL
DLLGQAAATQLARNISNSGAMQTYFAGETIPGDNLAYDADLSAWTEYIPYHFRPNYHG
VGTCSMMPKEMGGVVDNAARVYGVQGLRVIDGSIPPTQMSSHVMTVFYAMALKISD
AILEDYASMQ
 wherein X is selected from a cysteine or an amino acid including a thiol or selenol group, for example selenocysteine
SEQ ID NO: 4 (mutation at position 418) 
SNGIEASLLTDPKDVSGRTVDYIIAGGGLTGLTTAARLTENPNISVLVIESGSYESDRGP
IIEDLNAYGDIFGSSVDHAYETVELATNNQTALIRSGNGLGGSTLVNGGTWTRPHKAQ
VDSWETVFGNEGWNWDNVAAYSLQAERARAPNAKQIAAGHYFNASCHGVNGTVHA
GPRDTGDDYSPIVKALMSAVEDRGVPTKKDFGCGDPHGVSMFPNTLHEDQVRSDAA
REWLLPNYQRPNLQVLTGQYVGKVLLSQNGTTPRAVGVEFGTHKGNTHNVYAKHEV
LLAAGSAVSPTILEYSGIGMKSILEPLGIDTVVDLPVGLNLQDQTTATVRSRITSAGAGQ
GQAAWFATFNETFGDYSEKAHELLNTKLEQWAEEAVARGGFHNTTALLIQYENYRD
WIVNHNVAYSELFLDTXGVASFDVWDLLPFTRGYVHILDKDPYLHHFAYDPQYFLNEL
DLLGQAAATQLARNISNSGAMQTYFAGETIPGDNLAYDADLSAWTEYIPYHFRPNYHG
VGTCSMMPKEMGGVVDNAARVYGVQGLRVIDGSIPPTQMSSHVMTVFYAMALKISD
AILEDYASMQ
 wherein X is selected from a cysteine or an amino acid including a thiol or selenol group, for example selenocysteine
SEQ ID NO: 5 (mutation at position 446) 
SNGIEASLLTDPKDVSGRTVDYIIAGGGLTGLTTAARLTENPNISVLVIESGSYESDRGP
IIEDLNAYGDIFGSSVDHAYETVELATNNQTALIRSGNGLGGSTLVNGGTWTRPHKAQ
VDSWETVFGNEGWNWDNVAAYSLQAERARAPNAKQIAAGHYFNASCHGVNGTVHA
GPRDTGDDYSPIVKALMSAVEDRGVPTKKDFGCGDPHGVSMFPNTLHEDQVRSDAA
REWLLPNYQRPNLQVLTGQYVGKVLLSQNGTTPRAVGVEFGTHKGNTHNVYAKHEV
LLAAGSAVSPTILEYSGIGMKSILEPLGIDTVVDLPVGLNLQDQTTATVRSRITSAGAGQ
GQAAWFATFNETFGDYSEKAHELLNTKLEQWAEEAVARGGFHNTTALLIQYENYRD
WIVNHNVAYSELFLDTAGVASFDVWDLLPFTRGYVHILDKDPYLXHFAYDPQYFLNEL
DLLGQAAATQLARNISNSGAMQTYFAGETIPGDNLAYDADLSAWTEYIPYHFRPNYHG
 
VGTCSMMPKEMGGVVDNAARVYGVQGLRVIDGSIPPTQMSSHVMTVFYAMALKISD
AILEDYASMQ
 wherein X is selected from a cysteine or an amino acid including a thiol or selenol group, for example selenocysteine
SEQ ID NO: 6 (*A. niger* wild type GOx genes from GenBank. The signalling sequence is indicated in bold)
   >gi|2357|emb|CAA34197.1| Glucose oxidase protein form *A. niger* 
MQTLLVSSLVVSLAAALPHYIRSNGIEASLLTDPKDVSGRTVDYIIAGGGLTGLTTAAR
LTENPNISVLVIESGSYESDRGPIIEDLNAYGDIFGSSVDHAYETVELATNNQTALIRSG
NGLGGSTLVNGGTWTRPHKAQVDSWETVFGNEGWNWDNVAAYSLQAERARAPNA
KQIAAGHYFNASCHGVNGTVHAGPRDTGDDYSPIVKALMSAVEDRGVPTKKDFGCG
DPHGVSMFPNTLHEDQVRSDAAREWLLPNYQRPNLQVLTGQYVGKVLLSQNGTTPR
AVGVEFGTHKGNTHNVYAKHEVLLAAGSAVSPTILEYSGIGMKSILEPLGIDTVVDLPV
GLNLQDQTTATVRSRITSAGAGQGQAAWFATFNETFGDYSEKAHELLNTKLEQWAEE
AVARGGFHNTTALLIQYENYRDWIVNHNVAYSELFLDTAGVASFDVWDLLPFTRGYV
HILDKDPYLHHFAYDPQYFLNELDLLGQAAATQLARNISNSGAMQTYFAGETIPGDNL
AYDADLSAWTEYIPYHFRPNYHGVGTCSMMPKEMGGVVDNAARVYGVQGLRVIDGS
IPPTQMSSHVMTVFYAMALKISDAILEDYASMQ
SEQ ID NO: 7 (wild type GOx) genomic DNA sequence 
TCTAATGGTATTGAGGCATCTCTTTTGACTGACCCTAAGGACGTCTCAGGTCGTAC
CGTTGATTATATCATTGCTGGAGGTGGTTTGACCGGTCTTACTACTGCAGCTAGAC
TGACCGAAAACCCAAACATCTCCGTGTTGGTTATCGAATCCGGTTCCTATGAATCT
GACAGAGGTCCAATCATTGAGGACTTGAATGCTTATGGTGACATCTTCGGATCTTC
TGTTGACCACGCTTATGAAACTGTCGAGTTAGCTACTAACAACCAAACTGCATTGA
TCAGATCTGGAAATGGTTTGGGTGGTTCCACCCTTGTTAACGGTGGAACCTGGAC
CCGTCCTCACAAGGCTCAGGTCGATTCCTGGGAAACTGTTTTCGGTAACGAAGGA
TGGAATTGGGACAATGTTGCTGCTTACTCTCTTCAAGCTGAGAGAGCACGTGCTC
CAAACGCCAAGCAAATCGCTGCTGGACACTACTTCAACGCCTCATGCCACGGTGT
CAATGGAACTGTGCACGCTGGTCCTAGAGATACCGGAGACGACTATTCTCCTATT
GTTAAGGCTCTGATGTCTGCTGTTGAAGACAGAGGTGTTCCTACCAAGAAAGACT
TCGGTTGCGGTGATCCTCACGGTGTGTCCATGTTTCCTAACACCTTGCATGAGGA
CCAGGTGAGATCCGATGCAGCTAGAGAGTGGCTTCTTCCTAACTACCAACGTCCT
 
AACTTGCAGGTCTTGACTGGTCAGTACGTTGGAAAGGTTCTTTTGTCCCAGAATG
GTACTACCCCTCGTGCTGTGGGTGTTGAGTTCGGTACTCACAAGGGTAACACTCA
CAACGTTTACGCCAAGCACGAGGTGCTTTTGGCAGCTGGTTCCGCAGTTTCCCCT
ACTATTCTGGAGTACTCAGGTATTGGTATGAAGTCTATTCTTGAACCATTGGGTAT
TGATACTGTCGTTGACTTGCCAGTTGGTTTGAACTTGCAAGACCAAACCACTGCTA
CTGTTAGATCCAGAATCACTTCTGCTGGTGCTGGTCAAGGTCAAGCTGCTTGGTTT
GCCACTTTCAACGAAACTTTCGGTGACTATTCCGAAAAGGCTCACGAATTGTTGAA
CACCAAACTGGAACAGTGGGCTGAAGAGGCTGTCGCTAGAGGTGGTTTTCACAAC
ACCACCGCTTTGTTGATCCAGTACGAAAACTACAGAGACTGGATCGTTAACCACAA
CGTTGCCTACTCTGAATTGTTCCTTGACACTGCAGGTGTCGCTTCTTTTGACGTGT
GGGACTTGTTGCCTTTCACCAGAGGTTACGTTCACATCTTGGACAAGGACCCATA
CCTTCACCACTTCGCTTACGACCCTCAATACTTCCTTAACGAGCTGGACTTGCTTG
GACAAGCTGCTGCTACTCAATTGGCAAGAAACATCTCTAACTCCGGAGCCATGCA
GACCTACTTCGCTGGAGAAACTATCCCTGGTGACAACTTAGCTTACGATGCTGAC
CTTTCTGCATGGACCGAATACATTCCATACCACTTCCGTCCAAACTACCACGGAGT
GGGTACCTGTTCTATGATGCCAAAGGAAATGGGTGGAGTTGTTGACAATGCTGCT
CGTGTCTATGGTGTTCAAGGTCTTAGAGTTATCGATGGTTCCATCCCTCCTACTCA
AATGTCTTCACATGTGATGACCGTCTTCTACGCTATGGCCTTGAAGATTTCTGACG
CAATCTTGGAGGACTACGCATCTATGCAATAA
SEQ ID NO: 8 (K13C GOx) genomic DNA sequence 
TCTAATGGTATTGAGGCATCTCTTTTGACTGACCCTTGTGACGTCTCAGGTCGTAC
CGTTGATTATATCATTGCTGGAGGTGGTTTGACCGGTCTTACTACTGCAGCTAGAC
TGACCGAAAACCCAAACATCTCCGTGTTGGTTATCGAATCCGGTTCCTATGAATCT
GACAGAGGTCCAATCATTGAGGACTTGAATGCTTATGGTGACATCTTCGGATCTTC
TGTTGACCACGCTTATGAAACTGTCGAGTTAGCTACTAACAACCAAACTGCATTGA
TCAGATCTGGAAATGGTTTGGGTGGTTCCACCCTTGTTAACGGTGGAACCTGGAC
CCGTCCTCACAAGGCTCAGGTCGATTCCTGGGAAACTGTTTTCGGTAACGAAGGA
TGGAATTGGGACAATGTTGCTGCTTACTCTCTTCAAGCTGAGAGAGCACGTGCTC
CAAACGCCAAGCAAATCGCTGCTGGACACTACTTCAACGCCTCATGCCACGGTGT
CAATGGAACTGTGCACGCTGGTCCTAGAGATACCGGAGACGACTATTCTCCTATT
GTTAAGGCTCTGATGTCTGCTGTTGAAGACAGAGGTGTTCCTACCAAGAAAGACT
TCGGTTGCGGTGATCCTCACGGTGTGTCCATGTTTCCTAACACCTTGCATGAGGA
CCAGGTGAGATCCGATGCAGCTAGAGAGTGGCTTCTTCCTAACTACCAACGTCCT
AACTTGCAGGTCTTGACTGGTCAGTACGTTGGAAAGGTTCTTTTGTCCCAGAATG
GTACTACCCCTCGTGCTGTGGGTGTTGAGTTCGGTACTCACAAGGGTAACACTCA
 
CAACGTTTACGCCAAGCACGAGGTGCTTTTGGCAGCTGGTTCCGCAGTTTCCCCT
ACTATTCTGGAGTACTCAGGTATTGGTATGAAGTCTATTCTTGAACCATTGGGTAT
TGATACTGTCGTTGACTTGCCAGTTGGTTTGAACTTGCAAGACCAAACCACTGCTA
CTGTTAGATCCAGAATCACTTCTGCTGGTGCTGGTCAAGGTCAAGCTGCTTGGTTT
GCCACTTTCAACGAAACTTTCGGTGACTATTCCGAAAAGGCTCACGAATTGTTGAA
CACCAAACTGGAACAGTGGGCTGAAGAGGCTGTCGCTAGAGGTGGTTTTCACAAC
ACCACCGCTTTGTTGATCCAGTACGAAAACTACAGAGACTGGATCGTTAACCACAA
CGTTGCCTACTCTGAATTGTTCCTTGACACTGCAGGTGTCGCTTCTTTTGACGTGT
GGGACTTGTTGCCTTTCACCAGAGGTTACGTTCACATCTTGGACAAGGACCCATA
CCTTCACCACTTCGCTTACGACCCTCAATACTTCCTTAACGAGCTGGACTTGCTTG
GACAAGCTGCTGCTACTCAATTGGCAAGAAACATCTCTAACTCCGGAGCCATGCA
GACCTACTTCGCTGGAGAAACTATCCCTGGTGACAACTTAGCTTACGATGCTGAC
CTTTCTGCATGGACCGAATACATTCCATACCACTTCCGTCCAAACTACCACGGAGT
GGGTACCTGTTCTATGATGCCAAAGGAAATGGGTGGAGTTGTTGACAATGCTGCT
CGTGTCTATGGTGTTCAAGGTCTTAGAGTTATCGATGGTTCCATCCCTCCTACTCA
AATGTCTTCACATGTGATGACCGTCTTCTACGCTATGGCCTTGAAGATTTCTGACG
CAATCTTGGAGGACTACGCATCTATGCAATAA
SEQ ID NO: 9 (D70C GOx) genomic DNA sequence 
TCTAATGGTATTGAGGCATCTCTTTTGACTGACCCTAAGGACGTCTCAGGTCGTAC
CGTTGATTATATCATTGCTGGAGGTGGTTTGACCGGTCTTACTACTGCAGCTAGAC
TGACCGAAAACCCAAACATCTCCGTGTTGGTTATCGAATCCGGTTCCTATGAATCT
GACAGAGGTCCAATCATTGAGGACTTGAATGCTTATGGTTGTATCTTCGGATCTTC
TGTTGACCACGCTTATGAAACTGTCGAGTTAGCTACTAACAACCAAACTGCATTGA
TCAGATCTGGAAATGGTTTGGGTGGTTCCACCCTTGTTAACGGTGGAACCTGGAC
CCGTCCTCACAAGGCTCAGGTCGATTCCTGGGAAACTGTTTTCGGTAACGAAGGA
TGGAATTGGGACAATGTTGCTGCTTACTCTCTTCAAGCTGAGAGAGCACGTGCTC
CAAACGCCAAGCAAATCGCTGCTGGACACTACTTCAACGCCTCATGCCACGGTGT
CAATGGAACTGTGCACGCTGGTCCTAGAGATACCGGAGACGACTATTCTCCTATT
GTTAAGGCTCTGATGTCTGCTGTTGAAGACAGAGGTGTTCCTACCAAGAAAGACT
TCGGTTGCGGTGATCCTCACGGTGTGTCCATGTTTCCTAACACCTTGCATGAGGA
CCAGGTGAGATCCGATGCAGCTAGAGAGTGGCTTCTTCCTAACTACCAACGTCCT
AACTTGCAGGTCTTGACTGGTCAGTACGTTGGAAAGGTTCTTTTGTCCCAGAATG
GTACTACCCCTCGTGCTGTGGGTGTTGAGTTCGGTACTCACAAGGGTAACACTCA
CAACGTTTACGCCAAGCACGAGGTGCTTTTGGCAGCTGGTTCCGCAGTTTCCCCT
ACTATTCTGGAGTACTCAGGTATTGGTATGAAGTCTATTCTTGAACCATTGGGTAT
 
TGATACTGTCGTTGACTTGCCAGTTGGTTTGAACTTGCAAGACCAAACCACTGCTA
CTGTTAGATCCAGAATCACTTCTGCTGGTGCTGGTCAAGGTCAAGCTGCTTGGTTT
GCCACTTTCAACGAAACTTTCGGTGACTATTCCGAAAAGGCTCACGAATTGTTGAA
CACCAAACTGGAACAGTGGGCTGAAGAGGCTGTCGCTAGAGGTGGTTTTCACAAC
ACCACCGCTTTGTTGATCCAGTACGAAAACTACAGAGACTGGATCGTTAACCACAA
CGTTGCCTACTCTGAATTGTTCCTTGACACTGCAGGTGTCGCTTCTTTTGACGTGT
GGGACTTGTTGCCTTTCACCAGAGGTTACGTTCACATCTTGGACAAGGACCCATA
CCTTCACCACTTCGCTTACGACCCTCAATACTTCCTTAACGAGCTGGACTTGCTTG
GACAAGCTGCTGCTACTCAATTGGCAAGAAACATCTCTAACTCCGGAGCCATGCA
GACCTACTTCGCTGGAGAAACTATCCCTGGTGACAACTTAGCTTACGATGCTGAC
CTTTCTGCATGGACCGAATACATTCCATACCACTTCCGTCCAAACTACCACGGAGT
GGGTACCTGTTCTATGATGCCAAAGGAAATGGGTGGAGTTGTTGACAATGCTGCT
CGTGTCTATGGTGTTCAAGGTCTTAGAGTTATCGATGGTTCCATCCCTCCTACTCA
AATGTCTTCACATGTGATGACCGTCTTCTACGCTATGGCCTTGAAGATTTCTGACG
CAATCTTGGAGGACTACGCATCTATGCAATAA
SEQ ID NO: 10 (A418C GOx) genomic DNA sequence 
TCTAATGGTATTGAGGCATCTCTTTTGACTGACCCTAAGGACGTCTCAGGTCGTAC
CGTTGATTATATCATTGCTGGAGGTGGTTTGACCGGTCTTACTACTGCAGCTAGAC
TGACCGAAAACCCAAACATCTCCGTGTTGGTTATCGAATCCGGTTCCTATGAATCT
GACAGAGGTCCAATCATTGAGGACTTGAATGCTTATGGTGACATCTTCGGATCTTC
TGTTGACCACGCTTATGAAACTGTCGAGTTAGCTACTAACAACCAAACTGCATTGA
TCAGATCTGGAAATGGTTTGGGTGGTTCCACCCTTGTTAACGGTGGAACCTGGAC
CCGTCCTCACAAGGCTCAGGTCGATTCCTGGGAAACTGTTTTCGGTAACGAAGGA
TGGAATTGGGACAATGTTGCTGCTTACTCTCTTCAAGCTGAGAGAGCACGTGCTC
CAAACGCCAAGCAAATCGCTGCTGGACACTACTTCAACGCCTCATGCCACGGTGT
CAATGGAACTGTGCACGCTGGTCCTAGAGATACCGGAGACGACTATTCTCCTATT
GTTAAGGCTCTGATGTCTGCTGTTGAAGACAGAGGTGTTCCTACCAAGAAAGACT
TCGGTTGCGGTGATCCTCACGGTGTGTCCATGTTTCCTAACACCTTGCATGAGGA
CCAGGTGAGATCCGATGCAGCTAGAGAGTGGCTTCTTCCTAACTACCAACGTCCT
AACTTGCAGGTCTTGACTGGTCAGTACGTTGGAAAGGTTCTTTTGTCCCAGAATG
GTACTACCCCTCGTGCTGTGGGTGTTGAGTTCGGTACTCACAAGGGTAACACTCA
CAACGTTTACGCCAAGCACGAGGTGCTTTTGGCAGCTGGTTCCGCAGTTTCCCCT
ACTATTCTGGAGTACTCAGGTATTGGTATGAAGTCTATTCTTGAACCATTGGGTAT
TGATACTGTCGTTGACTTGCCAGTTGGTTTGAACTTGCAAGACCAAACCACTGCTA
CTGTTAGATCCAGAATCACTTCTGCTGGTGCTGGTCAAGGTCAAGCTGCTTGGTTT
 
GCCACTTTCAACGAAACTTTCGGTGACTATTCCGAAAAGGCTCACGAATTGTTGAA
CACCAAACTGGAACAGTGGGCTGAAGAGGCTGTCGCTAGAGGTGGTTTTCACAAC
ACCACCGCTTTGTTGATCCAGTACGAAAACTACAGAGACTGGATCGTTAACCACAA
CGTTGCCTACTCTGAATTGTTCCTTGACACTTGTGGTGTCGCTTCTTTTGACGTGT
GGGACTTGTTGCCTTTCACCAGAGGTTACGTTCACATCTTGGACAAGGACCCATA
CCTTCACCACTTCGCTTACGACCCTCAATACTTCCTTAACGAGCTGGACTTGCTTG
GACAAGCTGCTGCTACTCAATTGGCAAGAAACATCTCTAACTCCGGAGCCATGCA
GACCTACTTCGCTGGAGAAACTATCCCTGGTGACAACTTAGCTTACGATGCTGAC
CTTTCTGCATGGACCGAATACATTCCATACCACTTCCGTCCAAACTACCACGGAGT
GGGTACCTGTTCTATGATGCCAAAGGAAATGGGTGGAGTTGTTGACAATGCTGCT
CGTGTCTATGGTGTTCAAGGTCTTAGAGTTATCGATGGTTCCATCCCTCCTACTCA
AATGTCTTCACATGTGATGACCGTCTTCTACGCTATGGCCTTGAAGATTTCTGACG
CAATCTTGGAGGACTACGCATCTATGCAATAA
SEQ ID NO: 11 (H446C GOx) genomic DNA sequence

TCTAATGGTATTGAGGCATCTCTTTTGACTGACCCTAAGGACGTCTCAGGTCGTAC
CGTTGATTATATCATTGCTGGAGGTGGTTTGACCGGTCTTACTACTGCAGCTAGAC
TGACCGAAAACCCAAACATCTCCGTGTTGGTTATCGAATCCGGTTCCTATGAATCT
GACAGAGGTCCAATCATTGAGGACTTGAATGCTTATGGTGACATCTTCGGATCTTC
TGTTGACCACGCTTATGAAACTGTCGAGTTAGCTACTAACAACCAAACTGCATTGA
TCAGATCTGGAAATGGTTTGGGTGGTTCCACCCTTGTTAACGGTGGAACCTGGAC
CCGTCCTCACAAGGCTCAGGTCGATTCCTGGGAAACTGTTTTCGGTAACGAAGGA
TGGAATTGGGACAATGTTGCTGCTTACTCTCTTCAAGCTGAGAGAGCACGTGCTC
CAAACGCCAAGCAAATCGCTGCTGGACACTACTTCAACGCCTCATGCCACGGTGT
CAATGGAACTGTGCACGCTGGTCCTAGAGATACCGGAGACGACTATTCTCCTATT
GTTAAGGCTCTGATGTCTGCTGTTGAAGACAGAGGTGTTCCTACCAAGAAAGACT
TCGGTTGCGGTGATCCTCACGGTGTGTCCATGTTTCCTAACACCTTGCATGAGGA
CCAGGTGAGATCCGATGCAGCTAGAGAGTGGCTTCTTCCTAACTACCAACGTCCT
AACTTGCAGGTCTTGACTGGTCAGTACGTTGGAAAGGTTCTTTTGTCCCAGAATG
GTACTACCCCTCGTGCTGTGGGTGTTGAGTTCGGTACTCACAAGGGTAACACTCA
CAACGTTTACGCCAAGCACGAGGTGCTTTTGGCAGCTGGTTCCGCAGTTTCCCCT
ACTATTCTGGAGTACTCAGGTATTGGTATGAAGTCTATTCTTGAACCATTGGGTAT
TGATACTGTCGTTGACTTGCCAGTTGGTTTGAACTTGCAAGACCAAACCACTGCTA
CTGTTAGATCCAGAATCACTTCTGCTGGTGCTGGTCAAGGTCAAGCTGCTTGGTTT
GCCACTTTCAACGAAACTTTCGGTGACTATTCCGAAAAGGCTCACGAATTGTTGAA
CACCAAACTGGAACAGTGGGCTGAAGAGGCTGTCGCTAGAGGTGGTTTTCACAAC

ACCACCGCTTTGTTGATCCAGTACGAAAACTACAGAGACTGGATCGTTAACCACAA
CGTTGCCTACTCTGAATTGTTCCTTGACACTGCAGGTGTCGCTTCTTTTGACGTGT
GGGACTTGTTGCCTTTCACCAGAGGTTACGTTCACATCTTGGACAAGGACCCATA
CCTTTGTCACTTCGCTTACGACCCTCAATACTTCCTTAACGAGCTGGACTTGCTTG
GACAAGCTGCTGCTACTCAATTGGCAAGAAACATCTCTAACTCCGGAGCCATGCA
GACCTACTTCGCTGGAGAAACTATCCCTGGTGACAACTTAGCTTACGATGCTGAC
CTTTCTGCATGGACCGAATACATTCCATACCACTTCCGTCCAAACTACCACGGAGT
GGGTACCTGTTCTATGATGCCAAAGGAAATGGGTGGAGTTGTTGACAATGCTGCT
CGTGTCTATGGTGTTCAAGGTCTTAGAGTTATCGATGGTTCCATCCCTCCTACTCA
AATGTCTTCACATGTGATGACCGTCTTCTACGCTATGGCCTTGAAGATTTCTGACG
CAATCTTGGAGGACTACGCATCTATGCAATAA

Some preferred embodiments of the present invention are described in the numbered clauses below:
1. A sensor comprising a mutated biomolecule immobilised to a substrate; wherein the mutated biomolecule is immobilised to the substrate *via* a linker comprising a thiol reactive group and a substrate adherence group; wherein the biomolecule is mutated to introduce one or more surface accessible thiol moieties; and wherein the mutated biomolecule is connected to the linker *via* said one or more surface accessible thiol moieties on the biomolecule and said thiol reactive group on the linker.
2. A sensor comprising a biomolecule immobilised to a substrate *via* a linker, wherein the linker is connected to the biomolecule through a thiol linkage, and wherein the substrate is a material having optical or charge carrying properties.
3. The sensor of clause 2, wherein the thiol linkage is between one or more surface accessible thiol moieties on the biomolecule and thiol reactive group(s) on the linker.
4. The sensor according to clause 3, wherein the biomolecule comprises one or more mutations, wherein said one or more mutations introduce said one or more surface-exposed thiol moieties.
5. The sensor according to clause 1 or clause 4, wherein said mutation is a substitution, wherein preferable the substituted residue is selected from a cysteine, selenocysteine or a non-natural amino acid including a thiol group.
6. The sensor according to any preceding clause, wherein the biomolecule is glucose oxidase, concanavalin A, glucose binding protein, cholesterol oxidase or lactate oxidase.
7. The sensor according to any preceding clause, wherein the substrate is a carbon nanomaterial, preferably a carbon nanocarrier, single walled carbon nanotube (SWCNT), graphene, fullerene, multi-walled carbon nanotube (MWCNT) or single-walled carbon nanohorn, more preferably a single walled carbon nanotube (SWCNT).
8. The sensor according to any preceding clause, wherein the linker is a hydrophobic linker and/or the linker does not comprise DNA.
9. The sensor according to any preceding clause, wherein the biomolecule is mutated to introduce one or more surface accessible thiol moieties at a position or positions other than the C-terminus of the biomolecule.
10. The sensor according to any preceding clause, wherein the substrate comprises a plurality of biomolecules immobilised on its surface, and wherein the biomolecules are immobilised in an ordered manner.
11. A method of obtaining structured immobilisation of biomolecules on a substrate, comprising modifying the biomolecules to comprise one or more surface-exposed thiol moieties; attaching linking groups to said modified biomolecules *via* the surface-exposed thiol moieties and a thiol reactive group on the linking group; and adhereing the biomolecules to the substrate via a substrate adherence group on the linker;
   preferably wherein the substrate is a carbon nanomaterial, further preferably wherein the carbon nanomaterial is a carbon nanocarrier, single walled carbon nanotube (SWCNT), graphene, fullerene, multi-walled carbon nanotube (MWCNT) or single-walled carbon nanohorn, more preferably a single walled carbon nanotube (SWCNT).
12. A glucose oxidase polypeptide comprising one or more mutations, a carbohydrate oxidase polypeptide comprising one or more mutations or a lactate oxidase polypeptide comprising one or more mutations; wherein said one or more mutations introduces a site-selective thiol conjugation site selected to permit conjugation of the polypeptide to a linker comprising a thiol reactive group, preferably wherein the mutation is a substitution, wherein preferably the substituted residue is selected from a cysteine or a non-natural amino acid containing a thiol group.
13. The polypeptide of clause 12, wherein the sequence of glucose oxidase comprises the sequence defined in SEQ ID NO: 1 or a functional variant thereof and wherein the sequence further comprises a mutation at one or more of positions 13, 70, 418 and 446 in SEQ ID NO: 1, preferably wherein the sequence of glucose oxidase is selected from SEQ ID NO: 2, 3, 4 or 5 or a functional variant thereof, wherein X is selected from a cysteine or a non-natural amino acid containing a thiol group.
14. A method of producing a sensor, comprising:
   a) providing a biomolecule comprising one or more surface exposed thiol moieties;
   b) contacting the biomolecule with a linker comprising a thiol reactive group such that the linker covalently links to the biomolecule *via* the thiol reactive group;
   c) adhering the biomolecule-linker to a substrate, wherein the biomolecule-linker adheres *via* the linker.

## Claims

1. A glucose sensor comprising a mutated biomolecule immobilised to a substrate, wherein the mutated biomolecule is immobilised to the substrate via a linker comprising a thiol reactive group and a substrate adherence group, wherein the biomolecule is mutated to introduce one or more surface accessible thiol moieties, and wherein the mutated biomolecule is connected to the linker via said one or more surface accessible thiol moieties on the biomolecule and said thiol reactive group on the linker, and wherein the biomolecule is glucose oxidase, concanavalin A, or glucose binding protein.

2. A glucose sensor comprising a biomolecule immobilised to a substrate via a linker, wherein the linker is connected to the biomolecule through a thiol linkage, and wherein the substrate is a material having optical or charge carrying properties, and wherein the biomolecule is glucose oxidase, concanavalin A, or glucose binding protein.

3. The glucose sensor of claim 2, wherein the thiol linkage is between one or more surface accessible thiol moieties on the biomolecule and thiol reactive group(s) on the linker.

4. The glucose sensor according to claim 3, wherein the biomolecule comprises one or more mutations, wherein said one or more mutations introduce said one or more surface-exposed thiol moieties.

5. The glucose sensor according to claim 1 or claim 4, wherein said mutation is a substitution, wherein preferable the substituted residues is selected from a cysteine, selenocysteine or a non-natural amino acid including a thiol group.

6. The glucose sensor according to any preceding claim, wherein the substrate is a carbon nanomaterial, preferably a carbon nanocarrier, single walled carbon nanotube (SWCNT), graphene, fullerene, multi-walled carbon nanotube (MWCNT) or single-walled carbon nanohorn, more preferably a single walled carbon nanotube (SWCNT).

7. The glucose sensor according to any preceding claim, wherein the linker is a hydrophobic linker and/or the linker does not comprise DNA.

8. The glucose sensor according to any preceding claim, wherein the biomolecule is mutated to introduce one or more surface accessible thiol moieties at a position or positions other than the C-terminus.

9. The glucose sensor according to any preceding claim, wherein the substrate comprises a plurality of biomolecules immobilised on its surface, and wherein the biomolecules are immobilised in an ordered manner.

10. A method for obtaining structured immobilisation of biomolecules on a substrate, comprising modifying the biomolecules to comprise one or more surface-exposed thiol moieties, attaching linking groups to said modified biomolecules via the surface-exposed thiol moieties and a thiol reactive group on the linking group; and adhering the biomolecules to the substrate via a substrate adherence group on the linker; preferably wherein the substrate is a carbon nanomaterial, further preferably wherein the carbon nanomaterial is a carbon nanocarrier, single walled carbon nanotube (SWCNT), graphene, fullerene, multi-walled carbon nanotube (MWCNT) or singlewalled carbon nanhom, more preferably a single walled carbon nanotube (SWCNT), and wherein the biomolecules are selected from glucose oxidase, concanavalin A, or glucose binding protein.

11. A glucose oxidase polypeptide comprising one or more mutations, or a carbohydrate oxidase polypeptide comprising one or more mutations, wherein said one or more mutations introduces a site-selective thiol conjugation site selected to permit conjugation of the polypeptide to a linker comprising a thiol reactive group, preferably wherein the mutation is a substitution, wherein preferably the substituted residue is selected from a cysteine or non-natural amino acid containing a thiol group.

12. The polypeptide of claim 11, wherein the sequence of glucose oxidase comprises the sequence defined in SEQ ID NO: 1 or a functional variant thereof and the sequence further comprises a mutation at one or more of positions 13, 70, 418 and 446 in SEQ ID NO: 1, preferably wherein the sequence of glucose oxidase is selected from SEQ ID NO: 2, 3, 4, or 5 or a functional variant thereof, wherein X is selected from a cysteine or a non-natural amino acid containing a thiol group.

13. A method of producing a sensor comprising:
a) providing a biomolecule comprising one or more surface exposed thiol moieties;
b) contacting the biomolecule with a linker comprising a thiol reactive group such that the linker covalently links to the biomolecule via the thiol group;
c) adhering the biomolecule-linker to a substrate, wherein the biomolecule-linker adheres via the linker,
wherein the biomolecule is glucose oxidase, concanavalin A, or glucose binding protein.

14. A glucose sensor, comprising
- a glucose binding polypeptide having a mutation that introduces a surface accessible thiol moiety,
- a linker comprising a thiol-reactive moiety and an aromatic moiety, and
- a carbon nanomaterial having an optical and/or charge carrying property, wherein
- the glucose binding polypeptide is covalently linked to the linker via the surface accessible thiol moiety and the thiol-reactive moiety,
- the linker is non-covalently attached to the carbon nanomaterial via the aromatic moiety, and
- the glucose binding polypeptide is or comprises a glucose oxidase polypeptide according claim 11 to 12, a carbohydrate oxidase according to claim 11, concanavalin A or glucose binding protein.

15. The glucose sensor according to claim 14, wherein
- the carbon nanomaterial is a carbon nanotube, particularly a single-walled carbon nanotube, particularly having an optical property, or a multi-walled carbon nanotube, particularly having a charge carrying property, and/or
- the aromatic moiety is selected from benzyl, phenyl, naphtyl, anthracenyl, porphyrinyl, triphenylenyl, dibenzocyclooctynyl, pentacenyl and pyrenyl, and/or
- the thiol reactive moiety is maleimide group.
